# EUROPEAN PATENT APPLICATION

(11) **EP 4 534 677 A1**
(43) Date of publication of application: **09.04.2025**
(21) Application number: 23835598.6
(22) Date of filing: 06.07.2023
(51) Int. Cl.: C12N 15/62, A61K 35/17, A61K 35/28, A61K 48/00, A61P 25/00, C12N 5/10, C12N 15/12, C12N 15/13, C12N 15/55, C12N 15/63

(54) **NUECLEIC ACID MOLECULE, VECTOR, RECOMBINANT CELLS, AND DRUG FOR TREATING CENTRAL NERVOUS SYSTEM DISEASES**

(30) Priority: 08.07.2022 JP 2022110492
(71) Applicant: JCR Pharmaceuticals Co., Ltd., Ashiya-shi, Hyogo 659-0021 (JP)
(72) Inventor: KINOSHITA Masafumi, Kobe-shi, Hyogo 651-2241 (JP); TAKAGI Haruna, Kobe-shi, Hyogo 651-2241 (JP); SONODA Hiroyuki, Kobe-shi, Hyogo 651-2241 (JP); SHIMADA Yota, Tokyo 105-8461 (JP); KOBAYASHI Hiroshi, Tokyo 105-8461 (JP); OHASHI Toya, Tokyo 105-8461 (JP); HIGUCHI Takashi, Tokyo 105-8461 (JP); MATSUSHIMA Saki, Tokyo 105-8461 (JP)
(74) Representative: Boult Wade Tennant LLP
(86) International application number: PCT/JP2023/025156
(87) International publication number: WO 2024/010067

(57) **Abstract**

An object of the present invention is to provide a nucleic acid molecule, a vector, a recombinant cell, and a drug for treating a central nervous system disease, which easily migrate to the central nervous system. The nucleic acid molecule of the present invention contains a nucleotide sequence encoding a fusion protein, the fusion protein containing: an anti-transferrin receptor (TfR) antibody or an antigen-binding fragment thereof; and a protein to be brought into function in the central nervous system.

## Description

### Technical Field

The present invention relates to a nucleic acid molecule, a vector, recombinant cells, and a drug for treating central nervous system diseases.

### Background Art

Mucopolysaccharidosis type II (MPS II) is an X-linked lysosomal disease caused by deficiency of iduronate-2-sulfatase (IDS). The deficiency of IDS causes accumulation of a glycosaminoglycan (GAG) as a substrate thereof, whereby various symptoms such as central nervous symptoms, characteristic facial features, joint contracture, hepatosplenomegaly and valvular heart disease are systemically exhibited.

As treatments for MPS II, there are enzyme replacement therapy (ERT) and hematopoietic stem cell transplantation (HSCT) or bone marrow transplantation (BMT). However, conventional ERT, or HSCT or BMT has not achieved a sufficient effect on central nervous lesions, bone lesions, and the like (for example, Non Patent Literature 1). In 2021, central nervous system penetration type IDS and ERT utilizing intracerebroventricular administration of enzymes were approved in Japan. ERT has a high therapeutic effect, but requires weekly infusion and an extremely expensive drug, and thus establishment of a cheaper and effective treatment method is an important problem.

The present inventors examined pretreatment methods and transplantation rates of HSCT or BMT using MPS II mice, but none of them had an effect on the central nerve (Non Patent Literatures 4 and 5). Meanwhile, as a method other than ERT and HSCT or BMT, Non Patent Literatures 2 and 3 report the effect of hematopoietic stem cell gene therapy on MPS II mice.

The present inventors have succeeded in reducing GAG in the brain of MPS II model mice, which could not be achieved by ERT or HSCT, by expressing an IDS gene in hematopoietic stem cells using a (B6/MPS II) viral promoter (MND promoter: Moloney murine leukemia virus LTR/myeloproliferative sarcoma virus enhancer) in model mice (Non Patent Literature 6). The present inventors have also developed a lentiviral vector system for gene therapy of mucopolysaccharidosis type II (Patent Literature 1). The present inventors have further reported that the hematopoietic stem cell gene therapy subsequently ameliorates central nervous system lesions in a mouse GM1-gangliosidosis model (Non Patent Literature 7).

### Citation List

### Patent Literature

Patent Literature 1: Japanese Unexamined Patent Publication No. 2019-122371
Patent Literature 2: Japanese Unexamined Patent Publication No. 2018-134095
Patent Literature 3: WO 2018/124121
Patent Literature 4: Japanese Unexamined Patent Publication No. 2022-017258

### Non Patent Literature

Non Patent Literature 1: Akiyama K, et al., Mol Genet Metab. 2014, 111(2):139-146.
Non Patent Literature 2: Miwa S, et al., Mol Genet Metab. 2020, 130(4): 262-273
Non Patent Literature 3: Wada M, et al., Mol Ther Methods Clin Dev. 2020, 19:261-274
Non Patent Literature 4: Yokoi T, et al., Mol Genet Metab. 2016, 119(3): 232-238
Non Patent Literature 5: Yokoi K, et al., J Inherit Metab Dis. 2015, 38(2):333-340
Non Patent Literature 6: Wakabayashi T, et al., Hum Gene Ther. 2015, 26(6):357-366
Non Patent Literature 7: Tsunogai T, et al., Mol Ther Methods Clin Dev. 2022, 25:448-460
Non Patent Literature 8: Kyosen SO, et al. Gene Ther. 2010 Apr; 17(4):521-30
Non Patent Literature 9: Matsuda J, et al., Glycoconj J. 1997; 14(6):729-36
Non Patent Literature 10: Toya Ohashi et al., Mol Ther. 2012 Oct; 20(10):1924-31

### Summary of Invention

### Technical Problem

It is recognized that, in the IDS gene therapy targeting hematopoietic stem cells as disclosed in Non Patent Literature 6 and the like, the transplanted cells derived from the IDS gene-introduced hematopoietic stem cells migrate to the central nerve, whereby IDS is secreted also in the central nervous system and a certain therapeutic effect is exhibited. On the other hand, the IDS secreted into the blood outside the central nerve cannot pass through the blood-brain barrier, and the contribution to the therapeutic effect in the central nervous system is limited.

Therefore, an object of the present invention is to provide a nucleic acid molecule, a vector, a recombinant cell, and a drug for treating a central nervous system disease, which easily migrate to the central nervous system.

### Solution to Problem

The present inventors have found that, as a result of using the previously developed lentiviral vector system for gene therapy of mucopolysaccharidosis type II (Patent Literature 1) and a nucleic acid molecule capable of expressing a fusion protein of IDS and an antibody against a transferrin receptor (TfR), accumulation of GAG in the central nervous system as well as in the internal organs can be significantly reduced. Further, the present inventors have found that, in this nucleic acid molecule, the IDS can be replaced with another protein to be brought into function in the central nervous system, thereby making it possible to treat various central nervous system diseases, and have eventually completed the present invention.

That is, the present invention includes the following.
[1] A nucleic acid molecule comprising a nucleotide sequence encoding a fusion protein, the fusion protein comprising: an anti-transferrin receptor (TfR) antibody or an antigen-binding fragment thereof; and a protein to be brought into function in a central nervous system.
[2] The nucleic acid molecule according to [1], wherein the protein to be brought into function in the central nervous system is a lysosomal enzyme.
[3] The nucleic acid molecule according to [2], wherein the lysosomal enzyme is acid α-glucosidase (GAA) or β-galactosidase (GLB1).
[4] The nucleic acid molecule according to [1], wherein the protein to be brought into function in the central nervous system is iduronate-2-sulfatase (IDS).
[5] The nucleic acid molecule according to [1], wherein the protein to be brought into function in the central nervous system is a neurotrophic factor.
[6] The nucleic acid molecule according to [1], wherein the protein to be brought into function in the central nervous system is an antibody.
[7] A vector comprising the nucleic acid molecule according to any one of [1] to [6].
[8] The vector according to [7], wherein the vector stably expresses the fusion protein when the nucleic acid molecule is introduced into a host cell using the vector.
[9] The vector according to [8], wherein the vector is a lentiviral vector.
[10] A recombinant cell obtained by introducing the nucleic acid molecule into a host cell using the vector according to [7] or [8].
[11] The recombinant cell according to [10], wherein the host cell is a hematopoietic stem cell, a T cell, or a B cell.
[12] A drug for diagnosing, preventing or treating a central nervous system disease, the drug comprising the recombinant cell according to [10].
[13] A method for diagnosing, preventing, or treating a central nervous system disease, the method including transplanting the recombinant cell according to [10] into a subject in need thereof.
[14] Use of the recombinant cell according to [10] for diagnosing, preventing or treating a central nervous system disease.
[15] The recombinant cell according to [10] for use in diagnosis, prevention or treatment of a central nervous system disease.
[16] Use of the recombinant cell according to [10] in manufacture of a drug for diagnosing, preventing or treating a central nervous system disease.

### Advantageous Effects of Invention

The present invention provides a nucleic acid molecule, a vector, a recombinant cell, and a drug for treating a central nervous system disease such as a disease caused by IDS deficiency and a disease related to a protein to be brought into function in the central nervous system other than IDS. According to the present invention, the transplantation of a host cell, such as a hematopoietic stem cell, expressing a fusion protein of an anti-TfR antibody or an antigen-binding fragment thereof and a protein to be brought into function in the central nervous system such as IDS increases migration of the protein such as IDS to the central nervous system, thereby making it possible to treat a central nervous system disease such as mucopolysaccharidosis type II. In the present invention, since autologous hematopoietic stem cells of a patient can also be used, it is not necessary to search for a compatible donor as in conventional hematopoietic stem cell transplantation or the like which is allogenic transplantation, and the risk of graft versus host disease (GVHD) associated with transplantation and engraftment failure due to rejection is extremely low. In addition, since it is basically possible to maintain a sustained effect by one transplantation, the lifetime medical cost is expected to be inexpensive as compared with the enzyme replacement therapy, and the therapeutic effect is also high.

The present inventors have succeeded in allowing a protein to be brought into function in the central nervous system to migrate to the central nervous system by using a nucleic acid molecule containing a nucleotide sequence encoding a fusion protein containing an anti-TfR antibody or an antigen-binding fragment thereof and the protein to be brought into function in the central nervous system, and have proved for the first time that the protein actually exerts a therapeutic effect in the central nervous system. The migration of a recombinant cell itself, such as a hematopoietic stem cell, into the central nerve, into which the nucleic acid molecule of the present invention is introduced, is also conceivable. As described above, since both the recombinant cell and a fusion protein expressed and secreted by the cell (that is, a fusion protein of a therapeutic protein such as IDS and an anti-TfR antibody) migrate to the central nervous system, it is difficult to predict the dynamics of the fusion protein. Thus, it is difficult to predict whether a therapeutic effect is actually obtained. In addition, it is considered that most of the fusion protein migrates to the central tissue due to the presence of the anti-TfR antibody. Then, since a therapeutic effect in tissues other than the central nervous system cannot be expected, a treatment method involving allowing a therapeutic protein to migrate to the central nervous system has been avoided. However, surprisingly, the fusion protein of the present invention can also exert a certain therapeutic effect on tissues (for example, liver and the like) other than the central nervous system, as proved in Examples. Furthermore, it is believed that, since the fusion protein is large in molecular weight and also complex in molecule, the recombinant cell does not immediately attempt to produce the fusion protein that is large in molecular weight and complex. Therefore, a decrease in productivity of the fusion protein is expected, which makes prediction of the therapeutic effect more difficult. In view of these, the effect obtained by the invention is an unexpected remarkable effect.

### Brief Description of Drawings

FIG. 1 shows IDS activity in the plasma from 1 month to 6 months after transplantation of gene-introduced hematopoietic stem cells in Example 1.
FIG. 2 shows results of measurement of IDS activities and GAG accumulations in the liver and spleen 6 months after transplantation of gene-introduced hematopoietic stem cells in Example 1.
FIG. 3 shows results of measurement of IDS activities and GAG accumulations in the kidney and heart 6 months after transplantation of gene-introduced hematopoietic stem cells in Example 1.
FIG. 4 shows results of measurement of IDS activities and GAG accumulations in the cerebrum and cerebellum 6 months after transplantation of gene-introduced hematopoietic stem cells in Example 1.
FIG. 5 shows results of measurement of IDS activity in the plasma 4 weeks after transplantation of gene-introduced hematopoietic stem cells in Example 2.
FIG. 6 shows results of measurement of IDS activities and GAG accumulations in the liver and spleen 4 weeks after transplantation of gene-introduced hematopoietic stem cells in Example 2.
FIG. 7 shows results of measurement of IDS activities and GAG accumulations in the cerebrum and cerebellum 4 weeks after transplantation of gene-introduced hematopoietic stem cells in Example 2.
FIG. 8 shows results of measurement of IDS activity in the plasma 4 weeks after transplantation of gene-introduced T cells in Example 3.
FIG. 9 shows results of measurement of IDS activities and GAG accumulations in the cerebrum and cerebellum 4 weeks after transplantation of gene-introduced T cells in Example 3.
FIG. 10 shows results of measurement of GAA activity in gene-introduced HEK293T cells in Example 4.
FIG. 11 shows results of measurement of GAA activities in the serum, liver and spleen 4 weeks after transplantation of gene-introduced hematopoietic stem cells in Example 4.
FIG. 12 shows results of measurement of GAA activities in the heart, quadriceps femoris, diaphragm, and gastrocnemius muscle 4 weeks after transplantation of gene-introduced hematopoietic stem cells in Example 4.
FIG. 13 shows results of measurement of glycogen accumulations in the heart, quadriceps femoris, diaphragm, and gastrocnemius muscle 4 weeks after transplantation of gene-introduced hematopoietic stem cells in Example 4.
FIG. 14 shows results of measurement of GAA activities and glycogen accumulations in the cerebrum and cerebellum 4 weeks after transplantation of gene-introduced hematopoietic stem cells in Example 4.
FIG. 15 shows results of measurement of GLB1 activity in the serum 4 weeks after transplantation of gene-introduced hematopoietic stem cells in Example 5.
FIG. 16 shows results of measurement of GLB1 activities and GM1 accumulations in the cerebral cortex and cerebellum 4 weeks after transplantation of gene-introduced hematopoietic stem cells in Example 5.
FIG. 17 shows results of measurement of GLB1 activity and GM1 accumulation in the hippocampus 4 weeks after transplantation of gene-introduced hematopoietic stem cells in Example 5.

### Description of Embodiments

### [Nucleic Acid Molecule]

The nucleic acid molecule according to an embodiment of the present invention contains a nucleotide sequence encoding a fusion protein, the fusion protein containing: an anti-transferrin receptor (TfR) antibody or an antigen-binding fragment thereof; and a protein to be brought into function in the central nervous system. Preferably, the anti-TfR antibody is an anti-human TfR antibody, and the IDS is a human IDS.

### <Transferrin Receptor (TfR)>

The transferrin receptor (TfR) is a transmembrane protein that takes a conjugate of transferrin and iron (Fe) in blood into cells, and exists on the surface of vascular endothelial cells such as cerebral vascular endothelial cells. The anti-TfR antibody according to an embodiment of the present invention is an antibody that specifically binds to TfR and is thereby taken into a cell by TfR. At that time, IDS forming a fusion protein with the anti-TfR antibody is also taken into the cell together. Thus, the IDS that cannot pass through the blood-brain barrier (BBB) alone can be transported to brain tissue by TfR and show its physiological activity in the brain tissue. Therefore, the fusion protein of the anti-TfR antibody and the IDS can be used as a medicament to exert drug efficacy in the brain.

In the present specification, the origin of the transferrin receptor (TfR) is not limited, and it may be, for example, derived from human, or may be derived from mouse, rat, rabbit, horse, or a primate other than human, but is preferably derived from human. The human-derived TfR may be a wild-type TfR (protein: NP_001121620; gene: NM_001128148) or a mutated TfR. The mutated TfR is not particularly limited as long as the antibody against the mutated TfR is an antibody capable of binding also to the wild-type TfR, and may be, for example, a mutated TfR having enhanced physiological activity or antigenicity.

When an amino acid in the amino acid sequence of a protein having the physiological activity of the wild-type TfR is substituted with another amino acid in the mutated TfR, the number of amino acids to be substituted is preferably 1 to 20, more preferably 1 to 10, and still more preferably 1 to 3. When an amino acid of the wild-type TfR is deleted, the number of amino acids to be deleted is preferably 1 to 20, more preferably 1 to 10, and still more preferably 1 to 3. In addition, a mutation including a combination of the amino acid substitution and deletion can also be added. When an amino acid is added to the wild-type TfR, preferably 1 to 20, more preferably 1 to 10, and still more preferably 1 to 3 amino acids are added in the amino acid sequence of the protein or at the N-terminus or C-terminus. In addition, a mutation including a combination of the amino acid addition, substitution and deletion can also be added. The amino acid sequence of the protein to which the mutation is added shows preferably 80% or more identity, preferably 85% or more identity, more preferably 90% or more identity, still more preferably 95% or more identity, and even more preferably 98% or more identity with the amino acid sequence of the wild-type TfR.

In the present invention, the position and form (deletion, substitution, addition) of each mutation as compared with the wild-type TfR can be easily confirmed by alignment of the amino acid sequences of the wild-type and the mutated protein. In the present invention, the identity between the amino acid sequence of the wild-type TfR and the amino acid sequence of the mutated TfR can be easily calculated using a well-known homology calculation algorithm. Examples of such an algorithm include BLAST (Altschul SF. J Mol. Biol. 215. 403-10, (1990)), Pearson and Lipman's similarity search method (Proc. Natl. Acad. Sci. USA. 85. 2444 (1988)), and Smith and Waterman's local homology algorithm (Adv. Appl. Math. 2. 482-9 (1981)).

The substitutions of amino acids in the amino acid sequence of the above protein with other amino acids occurs, for example, within amino acid families that are relevant in their side chains and chemical properties of the amino acids. It is expected that the substitutions within such amino acid families will not result in significant changes in function of the protein (that is, which are conservative amino acid substitutions). Such amino acid families include, for example:
(1) aspartic acid and glutamic acid which are acidic amino acids,
(2) histidine, lysine, and arginine which are basic amino acids;
(3) phenylalanine, tyrosine, and tryptophan which are aromatic amine acids,
(4) serine and threonine which are amino acids having a hydroxyl group (hydroxyamino acids),
(5) methionine, alanine, valine, leucine, and isoleucine which are hydrophobic amino acids,
(6) cysteine, serine, threonine, asparagine, and glutamine which are neutral hydrophilic amino acids;
(7) glycine and proline, which are amino acids affecting the orientation of the peptide chain,
(8) asparagine and glutamine which are amide type amino acids (polar amino acids),
(9) alanine, leucine, isoleucine, and valine, which are aliphatic amino acids,
(10) alanine, glycine, serine, and threonine, which are amino acids with small side chains, and
(11) alanine and glycine, which are amino acids with particularly small side chains.

### <Anti-transferrin Receptor (TfR) Antibody>

The "antibody" as used herein means a protein immunoglobulin that specifically binds to an antigen. The immunoglobulin may be derived from any of commonly known isotypes, including, but not limited to, IgA, secretory IgA, IgG, IgE, and IgM. Generally, an antibody includes at least two heavy chains and two light chains interconnected by disulfide bonds. Each heavy chain includes a heavy chain variable region (VH) and a heavy chain constant region (CH), and the heavy chain constant region includes three constant domains, i.e., CH1, CH2, and CH3. Each light chain includes a light chain variable region (VL) and a light chain constant region, and the light chain constant region includes one constant domain, or CL. The VH and VL regions include framework regions (FRs) and complementarity determining regions (CDRs), and each include three CDRs and four FRs in the order of FR1, CDR1, FR2, CDR2, FR3, CDR3, and FR4 from the N-terminus to the C-terminus. The variable regions of the heavy and light chains include a binding domain that interacts with an antigen.

In the present specification, the antibody includes monoclonal antibodies, recombinantly generated antibodies, monospecific antibodies, multispecific antibodies (including bispecific antibodies), human antibodies, humanized antibodies, chimeric antibodies, synthetic antibodies, and tetrameric antibodies containing two heavy chain molecules and two light chain molecules. In the present specification, in addition to common tetrameric antibodies, a dimeric antibody, a single-chain antibody, a single-domain antibody, and the like described later are also included in the antibody. These antibodies are also referred to as antibody fragments (antigen-binding fragments).

The "human antibody" refers to an antibody entirely encoded by a human-derived gene. However, an antibody encoded by a gene obtained by mutating an original human gene for the purpose of, for example, increasing gene expression efficiency is also a human antibody. An antibody obtained by combining two or more genes encoding a human antibody and replacing a part of one human antibody with a part of another human antibody is also a human antibody. The human antibody has three complementarity determining regions (CDRs) of an immunoglobulin light chain and three complementarity determining regions (CDRs) of an immunoglobulin heavy chain. The three CDRs of the immunoglobulin light chain are referred to as CDR1, CDR2, and CDR3 in order from the N-terminal side. The three CDRs of the immunoglobulin heavy chain are referred to as CDR1, CDR2, and CDR3 in order from the N-terminal side. An antibody in which the antigen specificity, affinity, and the like of a human antibody are modified by replacing the CDR(s) of one human antibody with the CDR(s) of other human antibodies is also a human antibody.

In the present specification, an antibody in which a mutation such as substitution, deletion, or addition is added to the amino acid sequence of the original antibody by modifying the gene of the original human antibody is also referred to as a human antibody. When an amino acid in the amino acid sequence of the original antibody is substituted with another amino acid, the number of amino acids to be substituted is preferably 1 to 20, more preferably 1 to 10, still more preferably 1 to 5, and even more preferably 1 to 3. When an amino acid in the amino acid sequence of the original antibody is deleted, the number of amino acids to be deleted is preferably 1 to 20, more preferably 1 to 10, still more preferably 1 to 5, and even more preferably 1 to 3. In addition, an antibody to which a mutation including a combination of the amino acid substitution and deletion is added is also a human antibody. When an amino acid is added, preferably 1 to 20, more preferably 1 to 10, still more preferably 1 to 5, and even more preferably 1 to 3 amino acids are added into the amino acid sequence of the original antibody or on the N-terminal side or the C-terminal side. An antibody to which a mutation including a combination of the amino acid addition, substitution and deletion is added is also a human antibody. The amino acid sequence of the antibody to which the mutation is added shows preferably 80% or more identity, more preferably 90% or more identity, still more preferably 95% or more identity, and even more preferably 98% or more identity with the amino acid sequence of the original antibody. That is, in the present invention, the term "human-derived gene" includes not only the human-derived original gene but also a gene obtained by modifying the human-derived original gene.

The term "humanized antibody" as used herein refers to an antibody in which the amino acid sequence of a part of the variable region (for example, in particular all or some of the CDRs) is derived from a mammal other than human, and the other region is derived from human. Examples of the humanized antibody include an antibody produced by replacing the three complementarity determining regions (CDR) of the immunoglobulin light chain and the three complementarity determining regions (CDR) of the immunoglobulin heavy chain constituting a human antibody with the CDRs of other mammals. The biological species of other mammals from which the CDRs to be transplanted into the appropriate positions of the human antibody are derived is not particularly limited as long as it is a mammal other than human, but is preferably mouse, rat, rabbit, horse, or a primate other than human, is more preferably mouse and rat, and is, for example, mouse.

In the present specification, the case where the antibody is a human antibody or a humanized antibody will be described in detail below. The light chains of human antibodies and humanized antibodies include a λ chain and a κ chain. The light chain constituting the antibody may be either a λ chain or a κ chain. In addition, the heavy chains of human antibodies and humanized antibodies include a y chain, a µ chain, an α chain, a σ chain, and an ε chain, which correspond to IgG, IgM, IgA, IgD, and IgE, respectively. The heavy chain constituting the antibody may be any of a y chain, a µ chain, an α chain, a σ chain, and an ε chain, but is preferably a γ chain. Further, the y chain of the heavy chain of the antibody includes a γ1 chain, a y2 chain, a y3 chain, and a y4 chain, which correspond to IgG1, IgG2, IgG3, and IgG4, respectively. When the heavy chain constituting the antibody is a y chain, the y chain may be any of a γ1 chain, a y2 chain, a y3 chain, and a y4 chain, but is preferably a γ1 chain or a y4 chain. When the antibody is a humanized antibody or a human antibody and is IgG, the light chain of the antibody may be either a λ chain or a κ chain, and the heavy chain of the antibody may be any of a γ1 chain, a γ2 chain, a y3 chain, and a y4 chain, but is preferably a γ1 chain or a y4 chain. For example, one preferred aspect of the antibody includes an antibody in which the light chain is a λ chain and the heavy chain is a γ1 chain.

As used herein, the term "chimeric antibody" refers to an antibody in which fragments of two or more different antibodies derived from two or more different species are linked. In an embodiment, the anti-TfR antibody is a humanized anti-TfR antibody.

A chimeric antibody of a human antibody and an antibody of another mammal is an antibody in which a part of the human antibody is replaced by a part of the antibody of the mammal other than human. The antibody includes an Fc region, an Fab region, and a hinge portion described below. Specific examples of such a chimeric antibody include a chimeric antibody in which the Fc region is derived from a human antibody while the Fab region is derived from an antibody of another mammal. Conversely, those in which the Fc region is derived from other mammals while the Fab region is derived from a human antibody are also chimeric antibodies. The hinge portion may be derived from either a human antibody or an antibody of another mammal. The same applies to the humanized anti-TfR antibody.

It can also be said that a chimeric antibody (for example, humanized anti-TfR antibody) is composed of a variable region and a constant region. Other specific examples of the chimeric antibody include one in which the constant region (CH) of the heavy chain and the constant region (CL) of the light chain are derived from a human antibody, while the variable region (VH) of the heavy chain and the variable region (VL) of the light chain are derived from an antibody of another mammal, and, conversely, one in which the constant region (CH) of the heavy chain and the constant region (CL) of the light chain are derived from an antibody of another mammal, while the variable region (VH) of the heavy chain and the variable region (VL) of the light chain are derived from a human antibody. Here, the biological species of another mammal is not particularly limited as long as it is a mammal other than human, but is preferably mouse, rat, rabbit, horse, or a primate other than human, and is more preferably mouse.

A chimeric antibody of a human antibody and a mouse antibody is particularly referred to as a "human/mouse chimeric antibody". Examples of the human/mouse chimeric antibody include a chimeric antibody in which the Fc region is derived from a human antibody while the Fab region is derived from a mouse antibody, and, conversely, a chimeric antibody in which the Fc region is derived from a mouse antibody while the Fab region is derived from a human antibody. The hinge portion is derived from either a human antibody or a mouse antibody. Other specific examples of the human/mouse chimeric antibody include one in which the constant region (CH) of the heavy chain and the constant region (CL) of the light chain are derived from a human antibody, while the variable region (VH) of the heavy chain and the variable region (VL) of the light chain are derived from a mouse antibody, and, conversely, one in which the constant region (CH) of the heavy chain and the constant region (CL) of the light chain are derived from a mouse antibody, while the variable region (VH) of the heavy chain and the variable region (VL) of the light chain are derived from a human antibody.

The antibody originally has a basic structure composed of a total of four polypeptide chains of two immunoglobulin light chains and two immunoglobulin heavy chains. However, when the term "antibody" is used in the present invention, the "antibody" as used herein includes, in addition to tetrameric antibodies having this basic structure,
(1) a dimeric antibody composed of a total of two polypeptide chains of one immunoglobulin light chain and one immunoglobulin heavy chain,
(2) a single-chain antibody formed by binding a linker to the C-terminal side of an immunoglobulin light chain and further binding an immunoglobulin heavy chain to the C-terminal side thereof,
(3) a single-chain antibody formed by binding a linker to the C-terminal side of an immunoglobulin heavy chain and further binding an immunoglobulin light chain to the C-terminal side thereof,
(4) a single-chain antibody (scFv) formed by binding a linker to the C-terminal side of a variable region of an immunoglobulin heavy chain and further binding a variable region of an immunoglobulin light chain to the C-terminal side,
(5) a single-chain antibody (scFv) formed by binding a linker to the C-terminal side of a variable region of an immunoglobulin light chain and further binding a variable region of an immunoglobulin heavy chain to the C-terminal side,
(6) an antibody composed of an Fab region in which an Fc region is deleted from the basic structure of the antibody and an antibody composed of an Fab region and all or a part of a hinge portion (Fab, F(ab') and F(ab')2), and
(7) a single-domain antibody. Furthermore, an scFv obtained by binding a variable region of a light chain and a variable region of a heavy chain via a linker to form a single-chain antibody is also included in the antibody in the present specification.

In the present specification, the "linker" refers to, for example, a linker including a peptide chain in which a plurality of amino acids are bound by a peptide bond. A linker including such a peptide chain can also be referred to as a "peptide linker". The "linker" can also be rephrased as "linker sequence" in the context of the present specification. The N-terminus of the linker and the C-terminus of another protein are bound by a peptide bond, and the N-terminus of another protein is further bound to the C-terminus of the linker, whereby the two proteins form a conjugate via the linker.

In an embodiment of the present invention, Fab refers to a molecule in which one light chain including a variable region and a CL region (constant region of the light chain) and one heavy chain including a variable region and a CH1 region (portion 1 of the constant region of the heavy chain) are bound by a disulfide bond between cysteine residues present in each of the light chain and the heavy chain. In the Fab, the heavy chain may further include a part of the hinge portion in addition to the variable region and the CH1 region (portion 1 of the constant region of the heavy chain), but the hinge portion in this case lacks a cysteine residue that is present in the hinge portion and binds the heavy chains of the antibody to each other. In Fab, the light chain and the heavy chain are bound to each other by a disulfide bond formed between a cysteine residue present in the constant region (CL region) of the light chain and a cysteine residue present in the constant region (CH1 region) of the heavy chain or a hinge portion. The heavy chain forming Fab is referred to as an Fab heavy chain. The Fab is composed of one light chain and one heavy chain since it lacks a cysteine residue that is present in the hinge portion and binds the heavy chains of the antibody to each other. The light chain constituting the Fab includes a variable region and a CL region. The heavy chain constituting the Fab may be composed of a variable region and a CH1 region, or may include a part of the hinge portion in addition to the variable region and the CH1 region. However, in this case, the hinge portion is selected so as not to include a cysteine residue binding the two heavy chains to each other so that no disulfide bond is formed between the two heavy chains in the hinge portion. In the F(ab'), the heavy chain contains, in addition to the variable region and the CH1 region, all or a part of a hinge portion including a cysteine residue that binds the heavy chains to each other. The F(ab')2 refers to a molecule in which two F(ab')s are bound by a disulfide bond between cysteine residues present in their respective hinge portions. The heavy chain forming F(ab') or F(ab')2 is referred to as an Fab' heavy chain. In addition, a polymer such as a dimer or a trimer formed by binding a plurality of antibodies directly or via a linker is also an antibody. Furthermore, without being limited to these, any antibody that contains a part of an antibody molecule and has a property of specifically binding to an antigen is included in the "antibody" referred to in the present invention. That is, as used in the present invention, the "light chain" includes those derived from a light chain and having an amino acid sequence of all or a part of the variable region. In addition, the "heavy chain" includes those derived from a heavy chain and having an amino acid sequence of all or a part of the variable region. Therefore, as long as it has the amino acid sequence of all or a part of the variable region, for example, those in which the Fc region is deleted are also heavy chains.

Here, the Fc or Fc region refers to a region including a fragment composed of a CH2 region (portion 2 of the constant region of the heavy chain) and a CH3 region (portion 3 of the constant region of the heavy chain) in the antibody molecule.

Furthermore, the antibody in an embodiment of the present invention also includes:
(8) scFab, scF(ab'), and scF(ab')2 in which a light chain and a heavy chain constituting Fab, F(ab'), or F(ab')2 shown in (6) above are bound via a linker sequence to form a single-chain antibody. Here, in the scFab, scF(ab'), and scF(ab')2, a linker sequence may be bound to the C-terminal side of the light chain, and a heavy chain may be further bound to the C-terminal side thereof, or a linker may be bound to the C-terminal side of the heavy chain, and a light chain may be further bound to the C-terminal side thereof. Furthermore, an scFv obtained by binding a variable region of a light chain and a variable region of a heavy chain via a linker to form a single-chain antibody is also included in the antibody in the present invention. In the scFv, a linker sequence may be bound to the C-terminal side of the variable region of the light chain, and a variable region of the heavy chain may be further bound to the C-terminal side thereof, or a linker sequence may be bound to the C-terminal side of the variable region of the heavy chain, and a variable region of the light chain may be further bound to the C-terminal side thereof.

Furthermore, the term "antibody" as used herein includes, in addition to full-length antibodies and the antibodies shown in (1) to (8) above, any form of antigen-binding fragment (antibody fragment) in which a part of a full-length antibody is deleted, that is a broader concept including (1) to (8) above. The antigen-binding fragment includes a heavy chain antibody, a light chain antibody, VHH, VNAR, and those in which a part thereof is deleted.

The term "antigen-binding fragment" refers to a fragment of an antibody which retains at least a part of specific binding activity with an antigen, and may include an antigen complementarity determining region (CDR). Examples of the antigen-binding fragment include Fab, Fab', F(ab')2, a variable region (Fv), a single-chain antibody (scFv) in which a heavy chain variable region (VH) and a light chain variable region (VL) are linked by an appropriate linker, a diabody that is a dimer of a polypeptide including a heavy chain variable region (VH) and a light chain variable region (VL), a minibody that is a dimer in which a part of a constant region (CH3) is bound to a heavy chain (H chain) of scFv, and other low molecular weight antibodies. However, the antigen binding fragment is not limited to these molecules as long as it has binding ability to an antigen.

As used herein, the term "single-chain antibody" refers to a protein which is formed by binding a linker to the C-terminal side of an amino acid sequence containing all or a part of the variable region of an immunoglobulin light chain, and further binding an amino acid sequence containing all or a part of the variable region of an immunoglobulin heavy chain to the C-terminal side thereof, and is capable of specifically binding to a specific antigen. A protein which is formed by binding a linker to the C-terminal side of an amino acid sequence containing all or a part of the variable region of an immunoglobulin heavy chain, and further binding an amino acid sequence containing all or a part of the variable region of an immunoglobulin light chain to the C-terminal side thereof, and is capable of specifically binding to a specific antigen is also the "single-chain antibody" in the present invention. For example, the antibodies shown in (2) and (3) above are included in the single-chain antibody. In a single-chain antibody in which an immunoglobulin light chain is bound to the C-terminal side of an immunoglobulin heavy chain via a linker, the immunoglobulin heavy chain usually has an Fc region deleted. The variable region of the immunoglobulin light chain has three complementarity determining regions (CDRs) involved in the antigenic specificity of the antibody. Similarly, the variable region of the immunoglobulin heavy chain also has three CDRs. These CDRs are the main regions that determine the antigen specificity of the antibody. Therefore, the single-chain antibody preferably includes all three CDRs of an immunoglobulin heavy chain and all three CDRs of an immunoglobulin light chain. However, as long as the antigen-specific affinity of the antibody is maintained, a single-chain antibody in which one CDR or a plurality of CDRs is/are deleted can also be used.

In the single-chain antibody, the linker disposed between the light chain and the heavy chain of the immunoglobulin is preferably a peptide chain composed of 2 to 50, more preferably 8 to 50, still more preferably 10 to 30, even more preferably 12 to 18 or 15 to 25, for example, 15 or 25 amino acid residues. Such a linker is not limited in its amino acid sequence as long as the antibody in which both chains are linked by the linker retains affinity for an antigen, but is preferably composed of glycine alone or glycine and serine, and has, for example, an amino acid sequence Gly-Ser, an amino acid sequence Gly-Gly-Ser, an amino acid sequence Gly-Gly-Gly, Gly-Gly-Gly-Gly-Ser (SEQ ID NO: 9), Gly-Gly-Gly-Gly-Gly-Ser (SEQ ID NO: 10), Ser-Gly-Gly-Gly-Gly (SEQ ID NO: 11), or a sequence in which these amino acid sequences are repeated 2 to 10 times or 2 to 5 times. For example, when the variable region of the immunoglobulin heavy chain is bound to the C-terminal side of the amino acid sequence composed of the entire region of the variable region of the immunoglobulin light chain via a linker to form ScFV, a linker having the sequence of SEQ ID NO: 9 repeated three times is suitably used.

In an embodiment of the present invention, the single-domain antibody refers to an antibody having a property of specifically binding to an antigen in a single variable region. The single-domain antibody includes an antibody including a variable region composed only of a variable region of a heavy chain (heavy chain single-domain antibody) and an antibody including a variable region composed only of a variable region of a light chain (light chain single-domain antibody). VHH and VNAR are types of single-domain antibodies.

In an embodiment of the present invention, specific examples of the anti-TfR antibody include those described in, for example, Patent Literatures 2 to 4.

### <Protein to Be Brought into Function in Central Nervous System>

In the present specification, the "protein to be brought into function in the central nervous system" is a protein having physiological activity in the central nervous system, and is a protein required to be brought into function in the central nervous system. Here, the protein also includes a peptide, and may be, for example, a protein having 2 to 3000, 10 to 1500, or 20 to 1000 amino acid residues. The protein to be brought into function in the central nervous system is not particularly limited as long as it is a protein having physiological activity in the central nervous system, and examples thereof include lysosomal enzyme, neurotrophic factor, antibody, hormone, somatomedin, insulin, glucagon, cytokine, lymphokine, blood coagulation factor, fusion protein of antibody and other proteins, granulocyte macrophage colony stimulating factor (GM-CSF), granulocyte colony stimulating factor (G-CSF), macrophage colony stimulating factor (M-CSF), erythropoietin, darbepoetin, tissue plasminogen activator (t-PA), thrombomodulin, follicle stimulating hormone (FSH), gonadotropin releasing hormone (GnRH), gonadotropin, DNasel, thyroid stimulating hormone (TSH), nerve growth factor (NGF), ciliary neurotrophic factor (CNTF), glial cell line-derived neurotrophic factor (GDNF), neurotrophin 3, neurotrophin 4/5, neurotrophin 6, neuregulin 1, activin, basic fibroblast growth factor (bFGF), fibroblast growth factor 2 (FGF2), epidermal growth factor (EGF), vascular endothelial growth factor (VEGF), interferon α, interferon β, interferon y, interleukin 6, PD-1, PD-1 ligand, tumor necrosis factor α receptor (TNF-α receptor), an enzyme having an activity of degrading beta amyloid, etanercept, pegvisomant, metreleptin, abatacept, asfotase, and a GLP-1 receptor agonist, and any one of antibody medicaments.

As a suitable example when the protein to be brought into function in the central nervous system is a lysosomal enzyme, there are indicated α-L-iduronidase, glucocerebrosidase, β-galactosidase (GLB1), GM2 activating protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl CoAα-glucosaminide N-acetyltransferase, N-acetylglucosamine-6 sulfamine, acid ceramidase, amylo-1,6-glucosidase, sialidase, aspartyl glucosaminidase, palmitoyl protein thioesterase-1 (PPT-1), tripeptidyl peptidase-1 (TPP-1), hyaluronidase-1, CLN1, CLN2, acid α-glucosidase (GAA), and the like. When the protein to be brought into function in the central nervous system is a lysosomal enzyme, the lysosomal enzyme is more preferably β-galactosidase (GLB1) or acid α-glucosidase (GAA).

The protein to be brought into function in the central nervous system is preferably acid α-glucosidase (alfa-glucosidase; GAA), and GAA is a causative enzyme of muscle disease known as Pompe disease. When GAA is deficient, glycogen, a substrate of GAA, accumulates in tissues, thereby causing various symptoms.

In the present specification, the origin of GAA is not limited, and it may be, for example, derived from human, or may be derived from mouse, rat, rabbit, horse, or a primate other than human, but is preferably derived from human. The human-derived GAA may be a wild-type GAA (for example, protein: NP_000143 (Genbank); gene: NM_000152 (Genbank) or the like) or a mutated GAA. The amino acid or nucleotide sequence of the wild-type GAA may be changed by several amino acids or about several bases to several tens of bases depending on reference database, but is not particularly limited as long as a person skilled in the art can recognize the wild-type GAA. The mutated GAA is not particularly limited, and may be, for example, a mutated GAA having enhanced physiological activity. The description of the mutated TfR also applies to the mutated GAA. Other proteins to be brought into function in the central nervous system may be, for example, derived from human, or may be derived from mouse, rat, rabbit, horse, or a primate other than human, but is preferably derived from human. The protein may be a wild-type protein or a mutated protein.

The protein to be brought into function in the central nervous system is preferably β-galactosidase (beta-galactosidase; GLB1), and GLB1 is a causative enzyme of a genetic disease known as GM1 gangliosidosis. When GLB1 is deficient, GM1 ganglioside, a substrate of GLB1, accumulates in tissues, thereby causing various symptoms.

In the present specification, the origin of GLB1 is not limited, and it may be, for example, derived from human, or may be derived from mouse, rat, rabbit, horse, or a primate other than human, but is preferably derived from human. The human-derived GLB1 may be a wild-type GLB1 (for example, protein: AAA51823 (GenBank), NP_000395 (GenBank), P16278 (Uniprot); gene: NM_000404 (GenBank), M34423 (GenBank) or the like) or a mutated GLB1. The amino acid or nucleotide sequence of the wild-type GLB1 may be changed by several amino acids or about several bases to several tens of bases depending on reference database, but is not particularly limited as long as a person skilled in the art can recognize the wild-type GLB1. The mutated GLB1 is not particularly limited, and may be, for example, a mutated GLB1 having enhanced physiological activity. The description of the mutated TfR also applies to the mutated GLB1. Other proteins to be brought into function in the central nervous system may be, for example, derived from human, or may be derived from mouse, rat, rabbit, horse, or a primate other than human, but is preferably derived from human. The protein may be a wild-type protein or a mutated protein.

The protein to be brought into function in the central nervous system is preferably iduronate-2 sulfatase (IDS), and IDS is a causative enzyme of mucopolysaccharidosis type II known as Hunter syndrome. When IDS is deficient, glycosaminoglycan (GAG), a substrate for IDS, accumulates in tissues, thereby causing various symptoms.

In the present specification, the origin of IDS is not limited, and it may be, for example, derived from human, or may be derived from mouse, rat, rabbit, horse, or a primate other than human, but is preferably derived from human. The human-derived IDS may be a wild-type IDS (protein: NP_000193; gene: NM_000202) or a mutated IDS. The mutated IDS is not particularly limited, and may be, for example, a mutated IDS having enhanced physiological activity. The description of the mutated TfR also applies to the mutated IDS. Other proteins to be brought into function in the central nervous system may be, for example, derived from human, or may be derived from mouse, rat, rabbit, horse, or a primate other than human, but is preferably derived from human. The protein may be a wild-type protein or a mutated protein.

### <Fusion Protein>

The fusion protein of the present invention is a fusion protein of an anti-transferrin receptor (TfR) antibody or an antigen-binding fragment thereof and a protein to be brought into function in the central nervous system, which is capable of diagnosing, preventing, or treating a central nervous system disease. In the fusion protein, the anti-TfR antibody or antigen-binding fragment thereof and the protein to be brought into function in the central nervous system may be fused in this order or in the reverse order from the N-terminus to the C-terminus, and the anti-TfR antibody or antigen-binding fragment thereof is preferably present at the N-terminus. In addition, a peptide linker may be present between the anti-TfR antibody or antigen-binding fragment thereof and the protein to be brought into function in the central nervous system. The peptide linker is as described above.

The fusion protein of an embodiment of the present invention may contain a marker protein for detecting the fusion protein, and examples of the marker protein include GFP, EGFP, YFP, RFP, mCherry, and luciferase. The marker protein may be located at the N-terminus or C-terminus of the fusion protein or between the anti-TfR antibody or antigen-binding fragment thereof and the protein to be brought into function in the central nervous system.

The fusion protein of an embodiment of the present invention may contain a plurality of proteins to be brought into function in the central nervous system. In this case, the proteins to be brought into function in the central nervous system are fused functionally, preferably via a linker. The anti-TfR antibody or antigen-binding fragment thereof may be present at either the N-terminus or the C-terminus, but is preferably present at the N-terminus.

### <Nucleic Acid Molecule>

As used herein, the "nucleic acid molecule" refers to either DNA obtained by polymerizing deoxyribonucleotides by phosphodiester bonds or RNA obtained by polymerizing ribonucleotides by phosphodiester bonds.

When the "nucleic acid molecule" is DNA, the DNA may be single-stranded (single chain) or double-stranded with a complementary strand. When the DNA is single-stranded, the DNA may be (+)-stranded or (-)-stranded. The individual deoxyribonucleotides constituting DNA may be of a naturally occurring type or may be of a modified natural type as long as a gene encoding a protein contained in DNA can be translated into mRNA in a mammalian (particularly, human) cell. In an embodiment of the present invention, the individual deoxyribonucleotides constituting DNA may be of a naturally occurring type or may be of a modified natural type as long as a gene encoding a protein contained in DNA can be translated into mRNA and all or a part of DNA can be replicated in a mammalian (particularly, human) cell.

When the "nucleic acid molecule" is RNA, the RNA may be single-stranded (single chain) or double-stranded with a complementary strand. When the RNA is single-stranded, the RNA may be (+)-stranded or (-)-stranded. In an embodiment of the present invention, the individual ribonucleotides constituting RNA may be of a naturally occurring type or may be modified as long as a gene encoding a protein contained in RNA can be reverse-transcribed into DNA in a mammalian (particularly, human) cell. In an embodiment of the present invention, the individual ribonucleotides constituting RNA may be of a naturally occurring type or may be modified as long as a gene encoding a protein contained in RNA can be translated into a protein in a mammalian (particularly, human) cell. The modification of the ribonucleotide is performed, for example, in order to suppress the degradation of RNA by RNase and to enhance the intracellular stability of RNA.

Among the nucleic acid molecules encoding the fusion protein, the nucleic acid molecule of a portion encoding the anti-TfR antibody or antigen-binding fragment thereof is not particularly limited, and examples thereof include the DNA sequences described in Patent Literatures 2 to 4.

When the protein to be brought into function in the central nervous system is IDS, the nucleic acid molecule of a portion encoding IDS among the nucleic acid molecules encoding the fusion protein may be the IDS gene (NM_000202) or cDNA. Alternatively, it may be, for example, DNA shown in SEQ ID NO: 3 and subjected to codon optimization. When the protein to be brought into function in the central nervous system is GAA, the nucleic acid molecule of a portion encoding GAA among the nucleic acid molecules encoding the fusion protein may be the GAA gene (for example, NM_000152) or cDNA. Alternatively, it may be, for example, DNA shown in SEQ ID NO: 18. When the protein to be brought into function in the central nervous system is GLB1, the nucleic acid molecule of a portion encoding GLB 1 among the nucleic acid molecules encoding the fusion protein may be the GLB1 gene (for example, NM_000404, M34423) or cDNA. Alternatively, it may be, for example, DNA shown in SEQ ID NO: 24 and subjected to codon optimization.

In the fusion protein, when a peptide linker is present between the anti-TfR antibody or antigen-binding fragment thereof and the protein to be brought into function in the central nervous system, the nucleic acid molecule encoding the fusion protein also contains a nucleic acid encoding the peptide linker.

### <Vector>

The vector of the invention contains the nucleic acid molecule of the invention. The vector is preferably a vector capable of autonomously replicating in a host cell and/or a vector capable of integrating into a chromosome of a host cell, the vector being capable of transcribing the nucleic acid molecule, and stably expressing a fusion protein when the nucleic acid molecule is introduced into a host cell using the vector. Specific examples of the vector include a lentiviral vector, an adenovirus-associated vector, a retroviral vector, a DNA vector, an RNA vector, an adenoviral vector, a baculoviral vector, an Epstein-Barr viral vector, a papovaviral vector, a vaccinia viral vector, and a herpes simplex viral vector. Among them, a lentiviral vector and a retroviral vector are preferable from the viewpoint of stable expression in a host cell.

The lentivirus is a virus belonging to the genus Lentivirus in the subfamily Orthoretroviridae which is one of the subfamilies of the family Retroviridae, and has a single-stranded (+)-stranded RNA genome (ssRNA). The lentiviral genome contains gag (a region encoding a structural protein including a capsid protein), pol (a region encoding a group of enzymes including a reverse transcriptase), and env (a region encoding an envelope protein necessary for binding to a host cell) as essential genes, which are sandwiched between two LTRs (5'LTR and 3'LTR). In addition, the lentiviral genome contains Rev (a region encoding a protein having a function of binding to RRE (rev responsive element) present in viral RNA to transport viral RNA from nucleus to cytoplasm), tat (a region encoding a protein having a function of binding to TAR present in the 5'LTR to increase promoter activity of LTR), vif, vpr, vpu, nef, and the like as auxiliary genes.

The lentivirus is an enveloped virus and infects cells when the envelope fuses with the cell membrane. The lentivirus is an RNA virus, and a reverse transcriptase exists in the virion. After lentiviral infection, the reverse transcriptase replicates single-stranded plus-stranded DNA from a (+)-stranded RNA genome, and further synthesizes double-stranded DNA. A protein that is a component of the virion is expressed from the double-stranded DNA, and the (+)-stranded RNA genome is packaged into the protein, whereby the virion is proliferated. In an embodiment of the present invention, the lentiviral vector has been developed based on the genome of HIV-1, which is a type of lentivirus, but is not limited thereto.

A first generation lentiviral vector consists of three plasmids: a packaging plasmid, an Env plasmid, and an introduced plasmid. The packaging plasmid has respective genes of gag and pol under control of a CMV promoter or the like. The Env plasmid has an env gene under control of a CMV promoter. The introduced plasmid has 5'LTR, RRE, a gene encoding a desired protein under control of a CMV promoter, and 3'LTR. By introducing these plasmids into a host cell by a general transfection technique, a recombinant virion in which a nucleic acid molecule containing a foreign gene between the first LTR and the second LTR is packaged in a capsid protein is obtained. The packaging plasmid of the first generation lentiviral vector also contains the respective auxiliary genes rev, tat, vif, vpr, vpu, and nef derived from the virus. Here, the desired protein in the present invention is a fusion protein of a ligand and a protein having physiological activity. A promoter that controls a gene encoding the desired protein is a promoter other than the CMV promoter, and suitable examples thereof include those including an MND promoter, a phosphoglycerate kinase (PGK) promoter, a CD11b promoter, an SV40 early promoter, a human elongation factor-1α (EF-1α) promoter, a human ubiquitin C promoter, a CAG promoter (a cytomegalovirus enhancer and a tri-β-actin promoter, a hybrid promoter of rabbit β-globin polyA), a retroviral Rous sarcoma virus LTR promoter, a dihydrofolate reductase promoter, a β-actin promoter, a mouse albumin promoter, a human albumin promoter, and a human α-1 antitrypsin promoter. The MND promoter set forth in SEQ ID NO: 1 is preferably used.

As with the first generation, a second generation lentiviral vector also consists of three plasmids: a packaging plasmid, an Env plasmid (envelope plasmid), and an introduced plasmid. However, the respective auxiliary genes vif, vpr, vpu, and nef that are not essential genes have been deleted from the packaging plasmid.

In a third generation lentiviral vector (that is, a lentiviral vector system), the Rev contained in the packaging plasmid in the second generation is separated into an independent Rev plasmid. Also, tat has been deleted from the packaging plasmid. In addition, a U3 region within the 5'LTR of the introduced plasmid has been replaced with a CMV promoter.

The packaging plasmid encodes a viral gene other than the envelope and provides a protein for making viral particles in trans. Since the modified gene product is not essential for infection of non-dividing cells, the modified genes (vif, fpr, vpu, nef) are deleted. Since the modified gene product is not essential but important for replication of HIV-1 and is also deeply linked to the pathogenicity of HIV-1, the deletion thereof increases safety. Since tat is essential for replication of HIV-1, the deletion of tat from the packaging plasmid further increases safety. Since the packaging plasmid does not have a packaging signal (Ψ), RNA transcribed from this plasmid is not taken into the viral particles.

Rev acts on RNA after transcription, and selectively carries unspliced mRNA out of the nucleus, thereby translating the structural protein. In the third generation, the plasmid is separate from the packaging plasmid.

Since the envelope plasmid can only infect CD4 positive cells in the envelope of HIV-1, VSV-G (vesicular stomatitis virus G gylcoprotein) is used to broaden the host range. The receptor of VSV-G is considered to be a phospholipid, and, by using VSV-G as an envelope, membrane fusion between the viral particles and the cells occurs without depending on the receptor on the cell surface. Therefore, it is basically possible to infect any animal species and any cell tumor. In addition, VSV-G is physically strong, and the virus particles can be easily concentrated by ultracentrifugation.

In the vector, a gene of interest is incorporated between the LTRs (repetitive sequences that control transcription expression) at both ends including a packaging signal (Ψ). Further, it has a primer binding site essential for reverse transcription, and RNA transcribed from this plasmid is taken into the viral particles. The LTR promoter activity of HIV-1 is so weak in the absence of tat that an internal promoter is used for expression of the incorporated foreign gene. In an embodiment, the internal promoter may be a PGK, CD11b or MND promoter.

RRE (rev responsive element) is necessary for Rev to bind and for full-length viral genomic RNA to be efficiently transported from nucleus to cytoplasm. During reverse transcription, the retrovirus begins synthesis of the positive strand from the PPT (polypurine tract) sequence immediately upstream of the 3'LTR, whereas the lentivirus has the same sequence called cPPT (central polypurine tract) at another place in the central portion of the genome, also from which synthesis is performed, resulting in a triple-stranded structure of about 100 base pairs in the middle called DNA flap in the final double-stranded cDNA. It has been suggested that the cPPT sequence affects the efficiency of reverse transcription, and the incorporation of the cPPT sequence into a vector increases the gene introduction efficiency. WPRE (woodchuck hepatitis virus posttranscriptional regulatory element) is incorporated to increase expression efficiency. That is, WPRE is considered to have a role of enhancing the active transport of mRNA from nucleus to cytoplasm and the stability of mRNA in cytoplasm, and the incorporation of this sequence into a vector increases the titer and the expression efficiency of the transgene. Also, the deletion of an enhancer/promoter portion at U3 of the 3'LTR will result in both of the 3' and 5'LTRs having no promoter activity in a proviral state, and transcription of the entire genome from the 5'R region will not occur. As a result, the lentiviral vector corresponding to the strain deficient in HIV-1 proliferation capacity and the like serves as an SIN (self inactivating) vector satisfying the requirement for the position paper, "3. Those which do not have LTR promoter activity in the provirus and do not transcribe the entire HIV genome". In addition, by replacing U3 in the 5'LTR with the CMV promoter, tat dependency is eliminated, and tat can be deleted from the packaging plasmid.

As an example, a self-inactivating lentiviral pLVSIN-CMV Neo vector (manufactured by Takara Bio Inc.) can be used. Further, for example, pJLV1 obtained as follows is preferably used. The pJLV1 is obtained by, based on the pLVSIN-CMV Neo vector as described in Examples, replacing the U3 promoter region of the 5'LTR with a CMV promoter to form a hybrid LTR; reducing the virus-derived sequence downstream of a packaging signal and removing a portion from the internal promoter PCMVIE to the neomycin resistance gene; and inserting an MND promoter. The vector of the present invention can be obtained by functionally inserting a nucleic acid molecule encoding a fusion protein downstream of the vector.

Recombinant virions have infectivity and thus can be used to introduce foreign genes into cells, tissues, or living bodies. The foreign gene in the present invention is a nucleic acid molecule encoding a fusion protein. In a host cell or the like into which the nucleic acid molecule is introduced, a fusion protein is expressed from the nucleic acid molecule.

Retroviruses have a single-stranded (+)-stranded RNA genome (ssRNA). The viral genome encodes the respective genes gag (which encodes a structural protein including a capsid protein), pol (which encodes an enzyme group including a reverse transcriptase), env (which encodes an envelope protein necessary for binding to a host cell), and a packaging signal (Ψ), which are sandwiched between two long terminal repeats (LTRs, 5'LTR and 3'LTR). When the retrovirus infects a host cell, (+)-stranded RNA and a reverse transcriptase migrate into the cell and are reverse-transcribed into double-stranded DNA.

Retroviral vectors have been developed mainly based on murine leukemia viruses, and the viral genome has been divided in order to eliminate self-replication capacity while maintaining its infectivity, for the purpose of eliminating pathogenicity and increasing safety. The first generation retroviral vector consists of a packaging plasmid (viral genome without the packaging signal) and an introduced plasmid (packaging signal, a part of gag, both ends of a foreign gene sandwiched between 5'LTR and 3'LTR). Therefore, when homologous recombination occurs in the gag sequence portion commonly present in the packaging plasmid and the introduced plasmid, a self-replicating retrovirus, that is, a replication competent (RC) virus appears. In the second generation retroviral vector, the LTR on the 3' side of the first generation packaging plasmid is replaced with a polyA addition signal. As a result, in order to generate an RC virus, homologous recombination needs to occur simultaneously at two positions upstream of the gag sequence and the polyA addition signal, and the occurrence probability is very low, and safety is enhanced. The third generation is composed of three plasmids, and the packaging plasmid of the second generation is further divided into a plasmid encoding gag/pol and a plasmid encoding env. As a result, in order to generate an RC virus, homologous recombination needs to occur simultaneously at three locations, the occurrence probability is extremely low, and safety is further improved.

Generally, for the production of recombinant retroviral virions, the packaging plasmid and introduced plasmid are first introduced into a host cell by a common transfection technique. Then, the 5' and 3' LTRs and the region containing the gene encoding the desired protein placed between the two LTRs are replicated in the host cell, and the resulting single-stranded (+)-stranded RNA is packaged into the retroviral capsid protein to form a recombinant retroviral virion. The recombinant retroviral virions have infectivity and thus can be used to introduce foreign genes into cells, tissues, or living bodies. The foreign gene in the present invention is a nucleic acid molecule encoding a fusion protein. In a host cell or the like into which the nucleic acid molecule is introduced, a fusion protein is expressed from the nucleic acid molecule.

The method for producing a vector according to the present invention will be described. The vector is produced by a cloning operation in which an insert DNA fragment of interest (a nucleic acid molecule encoding an anti-TfR antibody or an antigen-binding fragment thereof and a nucleic acid molecule encoding a protein to be brought into function in the central nervous system) is placed on a vector of interest (pJLV1 plasmid). At this time, the nucleic acid molecule encoding an anti-TfR antibody or an antigen-binding fragment thereof and the nucleic acid molecule encoding a protein to be brought into function in the central nervous system may be prepared in a state of being functionally linked, or may be separately prepared. In a case where they are prepared separately, they are set in advance so as to be functionally linked, at the time when a restriction enzyme site or the like is cleaved and linked.

First, in the preparation of the insert DNA, the restriction enzyme cleavage site of the DNA fragment of interest is confirmed in advance, and then the restriction enzyme to be used for excision is selected according to the cloning site of the vector to be used. The restriction enzyme site is not particularly limited, and may be, for example, 5'-NotI GCGGCCGC/3'-XhoI CTCGAG.

Next, in preparation of the vector, the cloning site of the vector of interest (pJLV1 plasmid) is cleaved with the restriction enzyme to linearize the vector. Then, ligation of the insert DNA and the linearized plasmid is performed to produce a vector according to the present invention.

A plasmid system including the packaging plasmid, the Env plasmid, the Rev plasmid, and the introduced plasmid (vector), also referred to as the third generation lentiviral vector, has more than 1/3 of the HIV-1 genome deleted in the entire system, and is quite unlikely to produce wild-type HIV-1. Since the homologous region between the respective plasmids is also minimized, there is little possibility that a virus having autonomous proliferation capacity will be produced by homologous recombination.

Next, a use mode of the lentiviral vector will be described. Each of the constructed vectors is co-transfected with a third generation packaging plasmid, a Rev plasmid, and a VSV-G envelope plasmid into mass-cultured 293T cells, for example, and a culture solution of supernatant is recovered after 3 days. At this point, the culture solution contains virus particles, which are purified by ultracentrifugation and stored by dispensing at -80°C.

In an embodiment of the present invention, the nucleic acid molecule can be in a form of being encapsulated in a liposome, lipid nanoparticles (LNPs) or the like. The liposome refers to a spherical vesicle having a lipid bilayer, and is mainly composed of a phospholipid, particularly phosphatidylcholine. However, the present invention is not limited to this, and as long as a lipid bilayer is formed, the liposome may be added with other lipids such as egg yolk phosphatidylethanolamine. Since the cell membrane is mainly composed of a phospholipid bilayer membrane, the liposome has an advantage of excellent biocompatibility. The lipid nanoparticles refer to lipid-based particles having a diameter of 10 nm to 1000 nm, typically less than about 200 nm, and can have hydrophobic (lipophilic) molecules encapsulated therein, and contain biocompatible lipids such as triglycerides, diglycerides, monoglycerides, fatty acids, and steroids as main components. It is considered that when a gene encapsulated in a liposome or a lipid nanoparticle is administered into a living body, the gene is taken into a cell by direct fusion with the cell membrane of the cell, endocytosis, or the like, and then migrate to the nucleus to introduce the gene into the cell. The gene introduction method using a liposome or lipid nanoparticle is superior to the gene introduction using a viral vector in that the size of the gene to be introduced is not limited and that safety is high. The liposome, lipid nanoparticle, or the like having the nucleic acid molecule of the present invention encapsulated therein can be used for introducing a gene of a fusion protein of a ligand and a protein having physiological activity into a cell, a tissue, or a living body. In a cell or the like into which the gene is introduced, a fusion protein is expressed from the gene. As used herein, the "liposome, lipid nanoparticle, or the like" is intended to include, in addition to the liposomes and lipid nanoparticles described above, polymer nanoparticles, micelles, emulsions, nanoemulsions, microspheres, nanospheres, microcapsules, nanocapsules, dendrimers, nanogels, metal nanoparticles, and any other nano-microparticles that can be used as a drug delivery system (DDS).

In the present invention, the behaviors of a nucleic acid molecule introduced into a cell, a tissue, or a living body in the form of a vector, the form of being encapsulated in a recombinant viral virion, or the form of being encapsulated in a liposome, a lipid nanoparticle, or the like are exemplified in (1) to (9) below. However, the behaviors of the nucleic acid molecule is not limited to these.
(1) In an embodiment, the nucleic acid molecule is single-stranded (+)-stranded RNA. When the nucleic acid molecule is introduced into a cell, a gene encoding a fusion protein of a ligand contained in the nucleic acid molecule and a protein having physiological activity is translated to express the fusion protein.
(2) In an embodiment, the nucleic acid molecule is single-stranded (+)-stranded RNA. When introduced into a cell, the nucleic acid molecule is reverse-transcribed into single-stranded (+)-stranded DNA, and then the DNA is transcribed/translated to express the fusion protein.
(3) In an embodiment, the nucleic acid molecule is single-stranded (+)-stranded or (-)-stranded RNA. When introduced into a cell, the nucleic acid molecule is reverse-transcribed and then formed into double-stranded DNA, and then the DNA is transcribed/translated to express the fusion protein.
(4) In an embodiment, the nucleic acid molecule is single-stranded (+)-stranded or (-)-stranded RNA. When introduced into a cell, the nucleic acid molecule is reverse-transcribed and then formed into a double-stranded DNA. Then, the DNA undergoes random or homologous recombination with the genome of the host cell and is incorporated into the genome, and the incorporated DNA is transcribed and translated to express the fusion protein.
(5) In an embodiment, the nucleic acid molecule is single-stranded (+)-stranded DNA. When introduced into a cell, the nucleic acid molecule is transcribed/translated to express the fusion protein.
(6) In an embodiment, the nucleic acid molecule is single-stranded (+)-stranded DNA. When the nucleic acid molecule is introduced into a cell, double-stranded DNA is synthesized from the nucleic acid molecule. Then, the double-stranded DNA is transcribed/translated to express the fusion protein.
(7) In an embodiment, the nucleic acid molecule is single-stranded (+)-stranded DNA or (-)-stranded DNA. When the nucleic acid molecule is introduced into a cell, double-stranded DNA is synthesized from the nucleic acid molecule. Then, the DNA undergoes random or homologous recombination with the genome of the host cell and is incorporated into the genome, and the incorporated DNA is transcribed and translated to express the fusion protein.
(8) In an embodiment, the nucleic acid molecule is double-stranded DNA. When introduced into a cell, the nucleic acid molecule is transcribed/translated to express the fusion protein.
(9) In an embodiment, the nucleic acid molecule is double-stranded DNA. The DNA undergoes random or homologous recombination with the genome of the host cell and is incorporated into the genome, and the incorporated DNA is transcribed and translated to express the fusion protein.

The nucleic acid molecule in the form of a vector, the form of being encapsulated in a recombinant viral virion, or the form of being encapsulated in a liposome, a lipid nanoparticle, or the like can be introduced into a cell, a tissue, or a living body.

When introduced into a living body, the nucleic acid molecule is administered by a parenteral means such as subcutaneous injection, intramuscular injection, or intravenous injection in the form of a vector, in the form of being encapsulated in a recombinant viral virion, or in the form of being encapsulated in a liposome, a lipid nanoparticle, or the like.

The nucleic acid molecule in the form of vector, the form of being encapsulated in a recombinant viral virion, or the form of being encapsulated in a liposome, a lipid nanoparticle, or the like can be used as various medicaments.

### <Recombinant Cell>

The recombinant cell of the present invention is a recombinant cell obtained by introducing the vector of the present invention into a host cell. The type of the host cell is not particularly limited, and examples thereof include stem cells such as hematopoietic stem cells, mesenchymal stem cells, dental pulp-derived stem cells, embryonic stem cells, endothelial stem cells, mammary stem cells, intestinal stem cells, hepatic stem cells, pancreatic stem cells, neural stem cells, and iPS cells, and immune cells such as T cells, B cells, and NK cells.

The recombinant cell into which the nucleic acid molecule is introduced comes to express the fusion protein of the present invention, and thus can be transplanted into a patient for therapeutic purposes.

### [Drug]

The drug of the present invention is a drug for diagnosing, preventing or treating a central nervous system disease, the drug containing the recombinant cell of the present invention. Examples of the central nervous system disease include neurodegenerative diseases such as Alzheimer's disease, Parkinson's disease, and Huntington's disease; psychiatric disorders such as schizophrenia and depression; multiple sclerosis, amyotrophic lateral sclerosis, tumors of the central nervous system including brain tumors, lysosomal diseases associated with brain disorders, glycogen storage diseases, muscular dystrophy, cerebral ischemia, encephalitis diseases, prion diseases, and traumatic central nervous system disorders. In addition, diseases caused by IDS deficiency, Gaucher's disease, GM1-gangliosidosis type 1 to 3, GM2-gangliosidosis AB variant, Sandhoff's disease and Tay-Sachs disease, Sandhoff's disease, I-cell disease, α-mannosidosis, β-mannosidosis, Krabbe disease, Gaucher disease-like storage disease, metachromatic leukodystrophy, fucosidosis, aspartyl-glucosaminuria, Schindler disease, Kawasaki disease, Niemann-Pick disease, Fabry disease, Sly syndrome, Sanfilippo syndrome, Hurler syndrome, Farber's disease, Cori disease (Forbes-Cori disease), sialidase deficiency, neuronal ceroid lipofuscinosis, Santavuori-Haltia disease, neuronal ceroid lipofuscinosis, Jansky-Bielschowsky disease, hyaluronidase deficiency, Pompe disease, and Batten disease. Among them, it is preferably a disease caused by IDS deficiency, GM1-gangliosidosis type 1 to 3, or Pompe disease. Examples of the disease caused by IDS deficiency include mucopolysaccharidosis type II and Hunter syndrome, and mucopolysaccharidosis type II is particularly preferable.

The drug of an embodiment contains an effective amount of the above recombinant cells, and the effective amount may vary depending on the administration route, administration interval, body weight and age, but is preferably, for example, 10⁴ to 10¹⁰ cells, preferably 10⁶ to 10⁹ cells, and more preferably 10⁷ to 10⁸ cells.

In addition to the recombinant cell, the drug of an embodiment may contain a carrier that can preserve cells and is pharmaceutically acceptable. As such a carrier, a physiological aqueous solvent (saline, buffer, serum-free medium, etc.) can be used. If necessary, a commonly used preservative, stabilizer, reducing agent, isotonizing agent, or the like may be blended in the drug.

### [Method]

The method of the present invention is a method for diagnosing, preventing, or treating a central nervous system disease, including transplanting the recombinant cell of the present invention into a subject in need thereof. The subject may be a patient having a central nervous system disease, particularly a patient with a disease caused by IDS deficiency, GM1-gangliosidosis type 1 to 3, or Pompe disease.

Examples of the transplantation method include a method in which a cell fluid containing an effective amount of the recombinant cell is intravenously administered to a subject.

When the recombinant cell is a hematopoietic stem cell, it can engraft in the bone marrow 14 to 28 days after transplantation, and the engrafted recombinant cell can differentiate into microglia-like cells in addition to various blood cells while self-proliferating, and engraft in the central nervous system. Thus, the recombinant cell can express the protein to be brought into function in the central nervous system continuously in various tissues including central nervous tissue.

### [Use]

The use of the present invention is use of the recombinant cell of the present invention for diagnosing, preventing or treating a central nervous system disease. The use of the present invention is also use of the recombinant cell of the present invention in manufacture of a drug for diagnosing, preventing or treating a central nervous system disease. The present invention also provides the recombinant cell of the present invention for use in diagnosis, prevention or treatment of a central nervous system disease.

### Examples

### [Example 1: Hematopoietic Stem Cell Gene Therapy Using Anti-mouse TfR Antibody-Human IDS]

### <Mouse>

Female C57BL/6 mice (GarciaAR, et al. J Inherit Metab Dis. 2007; 30(6):924-34.) having mouse IDS gene deficiency heterozygously and male normal C57BL/6 mice were crossed, and mice having IDS deficiency hemizygously were selected from the created males by PCR analysis. The male mice having IDS deficiency hemizygously were defined as MPS II mice, and male mice having wild-type IDS served as normal controls.

### <Construction of Lentiviral Vector>

pJLV1-IDS (Patent Literature 1: CMV hybrid 5'LTR-Ψ-RRE-cPPT-MND-IDS cDNA-WPREmut9-3'LTR (SEQ ID NO: 2)) carrying human IDS (GenBank: NG_011900) immediately below an MND promoter (DNA: SEQ ID NO: 1) of a pJLV1 plasmid (SEQ ID NO: 12), which is a self-inactivating lentiviral vector, and pJLV1-mTfR-IDS carrying an anti-mouse TfR antibody (mTfR)-IDS similarly immediately below the MND promoter were constructed.

### (1) Construction of pJLV1-IDS

The lentiviral vector pJLV1-IDS was constructed based on the pLVSIN-CMV Neo vector (Takara Bio Inc.). Specifically, the U3 promoter region of the 5'LTR was replaced with a CMV promoter to form a hybrid LTR. The virus-derived sequence downstream of a packaging signal was reduced, and a portion from the internal promoter PCMVIE to the neomycin resistance gene was removed. An MND promoter was inserted. A DNA fragment (SEQ ID NO: 3) encoding human IDS (SEQ ID NO: 4) and subjected to codon optimization was placed downstream of the MND promoter, and a NotI site and a XhoI site were inserted as restriction enzyme sites upstream and downstream of the DNA fragment. Furthermore, downstream of the XhoI site, a modified WPRE sequence (one adenine base was added at position 417) and 3' SIN LTR obtained by inserting a cHS4 core insulator sequence into the 3'LTR having a U3 region deleted were placed to obtain pJLV1-IDS having a nucleotide sequence of SEQ ID NO: 2.

### (2) Construction of pJLV1-mTfR-IDS

A vector pJLV1-mTfR-IDS (SEQ ID NO: 13) was produced by a cloning operation in which a DNA fragment (SEQ ID NO: 7) encoding a fusion protein (mTfR-IDS, SEQ ID NO: 8) functionally fused with human IDS (SEQ ID NO: 4) via a linker at the 3' terminus of an anti-mouse TfR antibody (anti-mouse TfR_scFV, SEQ ID NO: 6; codon optimized DNA: SEQ ID NO: 5) and subjected to codon optimization was placed on the vector pJLV1.

Specifically, DNA encoding mTfR-IDS was artificially synthesized in a state in which an EcoRI site was added to the 5' side and a HindIII site was added to the 3' side, and treated with a restriction enzyme. Next, pCMV-Script (Agilent Technologies) was cut with EcoRI and HindIII, and pCMV-Script-mTfR-IDS was prepared using Quick ligation kit (New England Biolab). Thereafter, pCMV-Script-mTfR-IDS and pJLV1-IDS were cleaved with the restriction enzymes NotI and XhoI, mTfR-IDS and pJLV1 plasmid were excised, and ligation thereof was performed using Quick ligation kit to produce pJLV1-mTfR-IDS (SEQ ID NO: 13).

### (4) Viral Packaging

Lentiviral packaging was performed using pCAG-HIVgp as a packaging plasmid and pCMV-VSV-G-RSV-Rev as a VSV-G/Rev plasmid, in addition to the self-inactivating (SIN) lentiviral vector (pJLV1-IDS or pJLV1-mTfR-IDS) produced in the above (1) or (2), by partially modifying a standard protocol (for example, the protocol described in Patent Literature 1).

Specifically, for HEK293T cells cultured to be 70 to 80% confluent on a Poly-L-Lysine-treated 245 mm × 245 mm dish,
each of plasmid DNAs:
packaging plasmid (pCAG-HIVgp): 30 µg
VSV-G, Rev plasmid (pCMV-VSV-G-RSV-Rev): 30 µg, and
SIN lentiviral vector: 60 µg
was subjected to transfection with 2.5 M CaCl₂ and cultured in a 5% CO₂ incubator at 37°C for 16 to 20 hours.

The culture supernatant was removed by suction, washed with PBS, replaced with 60 ml of a DMEM medium, and then cultured in the 5% CO₂ incubator at 37°C for about 48 hours. Thereafter, the culture supernatant was recovered, passed through a 0.45 µm filter, and then concentrated using Centricon Plus-70 (100 K).

The concentrated virus solution was diluted with PBS to the upper limit of a centrifuge tube, and ultracentrifugation was performed at 70,000 × g and 4°C for 1.5 hours. The centrifugal supernatant was removed, pellets were suspended in 1 ml of PBS, and the suspension was transferred to a 50 ml centrifuge tube and vortexed for 3 hours.

The vortexed suspension was centrifuged at 5000 rpm for 1 minute and the supernatant was pipetted into a screw capped tube and stored at - 80°C.

The titer of the lentivirus prepared was measured using Quick Titer HIV Lentivirus Quantitation Kit (Cell Biolabs, San Diego, CA), which is an ELISA for p24 protein. The titers were: 1.6 × 10⁹ IU/ml for pJLV1-IDS and 1.9 × 10⁹ IU/ml for pJLV1-mTfR-IDS.

### <Production and Transplantation of Gene-introduced Hematopoietic Stem Cell>

Bone marrow cells were collected from the femur of 2-month-old MPS II mice, and mouse hematopoietic stem cells were separated using Lineage Cell Depletion Kit(Miltenyi Biotec, Bergisch Gladbach, Germany). The obtained lentivirus was gene-introduced (infected) into the separated mouse hematopoietic stem cells. Lentiviral infection of the mouse hematopoietic stem cells was performed according to the method of the previous literature and at a multiplicity of infection (MOI = 50) (Non Patent Literature 2). Specifically, it was performed using 1 × 10⁸ IU of lentivirus per 2 × 10⁶ mouse hematopoietic stem cells (the number of cells for transplantation per recipient).

Transplantation of the gene-introduced hematopoietic stem cells was also performed according to the previously reported literature (Non Patent Literature 2). The gene-introduced hematopoietic stem cells were administered from the tail vein to the 2-month-old MPS II mice that had been irradiated with a lethal dose of 9 Gy using an X-ray irradiation system MBR-1520-R (Hitachi Power Solutions). Plasma was collected every month after administration, and liver, spleen, kidney, heart, cerebrum, and cerebellum were collected after 6 months. Untransplanted wild-type (WT) C57BL/6 mice and MPS II mice (MPS II) were used as control mice.

### <IDS Activity Measurement and Glycosaminoglycan (GAG) Measurement>

According to the previously reported literature (Non Patent Literature 2), the above tissues were extracted to measure IDS activity and GAG. Protein quantification was performed using a DC protein assay kit (Bio-Rad), IDS activity measurement was performed using 4-methylumbelliferone α-L-idopyranosiduronic acid 2-sulfate(Carbosynth, Berkshire, UK) as a fluorescent substrate, and GAG measurement was performed using a triple quadrupole high-performance liquid chromatograph mass spectrometer LCMS-8040 (Shimadzu Corporation).

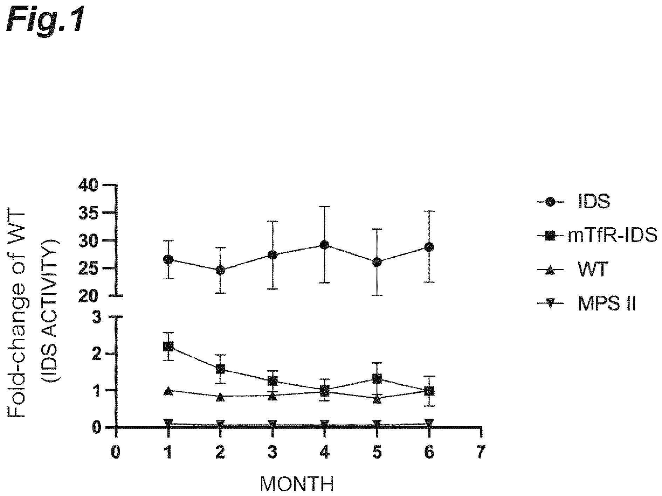

The IDS activity in plasma from 1 month to 6 months after transplantation is shown in FIG. 1. The IDS activity in FIG. 1 is a relative activity when the IDS activity in the wild-type mice (WT) is 1. From FIG. 1, it was found that, in the MPS II mice (IDS in FIG. 1) into which the lentivirus including pJLV1-IDS was introduced, the activity about 25 times higher than that of WT was maintained over 6 months after transplantation. In addition, the IDS activity in the MPS II mice (mTfR-IDS in FIG. 1) into which the lentivirus including pJLV1-mTfR-IDS was introduced was observed to be equal to or higher than that in WT, and was found to be comparable to that in WT even at 6 months. On the other hand, the IDS activity was hardly observed in the MPS II mice. In addition, the mTfR-IDS mice had about 1/30 of the IDS activity in the plasma of the IDS mice, suggesting that the amount of IDS secreted per cell in the mTfR-IDS mice is smaller than that in the IDS mice.

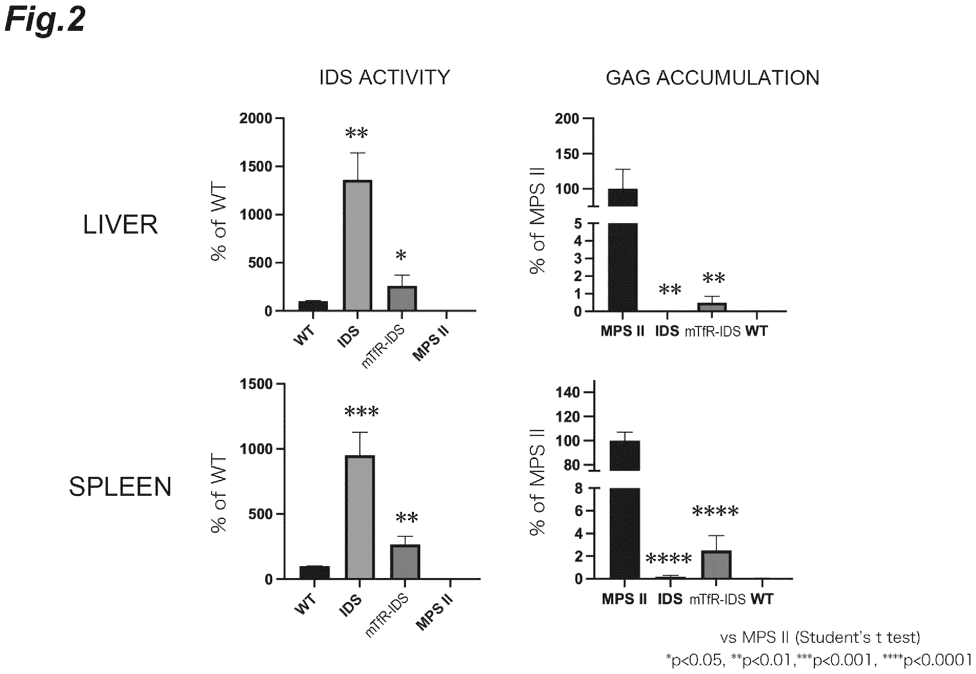

The results of measurement of the IDS activities and GAG accumulations in the liver and spleen 6 months after transplantation are shown in FIG. 2. The IDS activity is a relative activity (%) when the IDS activity in the wild-type mice (WT) is 100, and the GAG accumulation is a relative amount (%) when the amount of GAG accumulated in the MPS II mice (MPS II) is 100. From FIG. 2, both the IDS and mTfR-IDS mice showed IDS activities in the liver and spleen higher than those of the wild type, and also showed significantly reduced GAG accumulations as compared to those of MPS II.

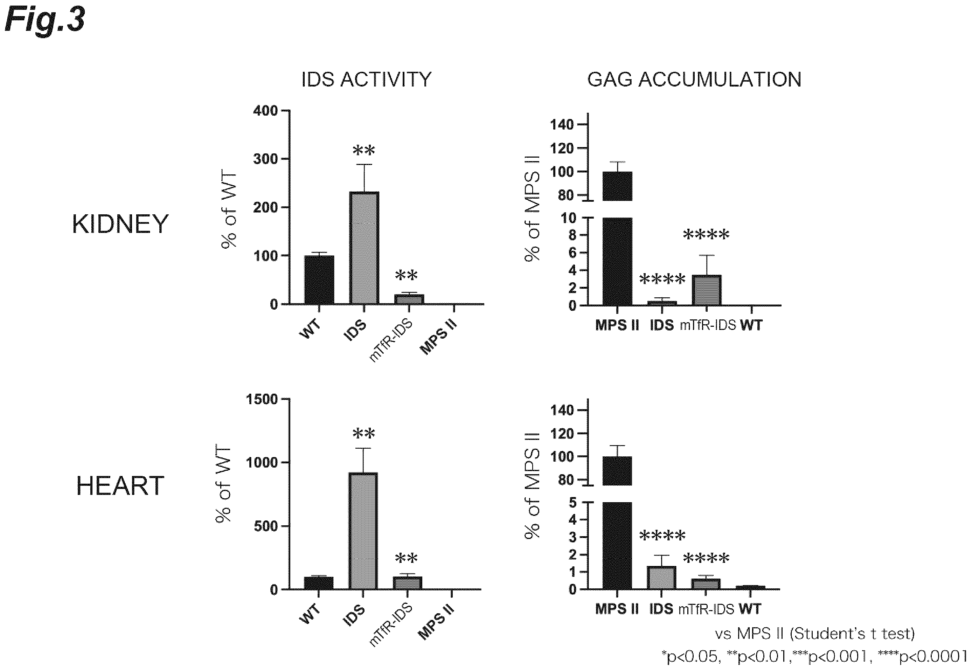

The results of measurement of the IDS activities and GAG accumulations in the kidney and heart 6 months after transplantation are shown in FIG. 3. From FIG. 3, both the IDS mice and the mTfR-IDS mice showed IDS activity in the heart equal to or higher than that of the wild type (WT), but only the IDS mice showed IDS activity in the kidney higher than that of the wild type. However, the GAG accumulations in the heart and kidney were significantly reduced in both the IDS and mTfR-IDS mice.

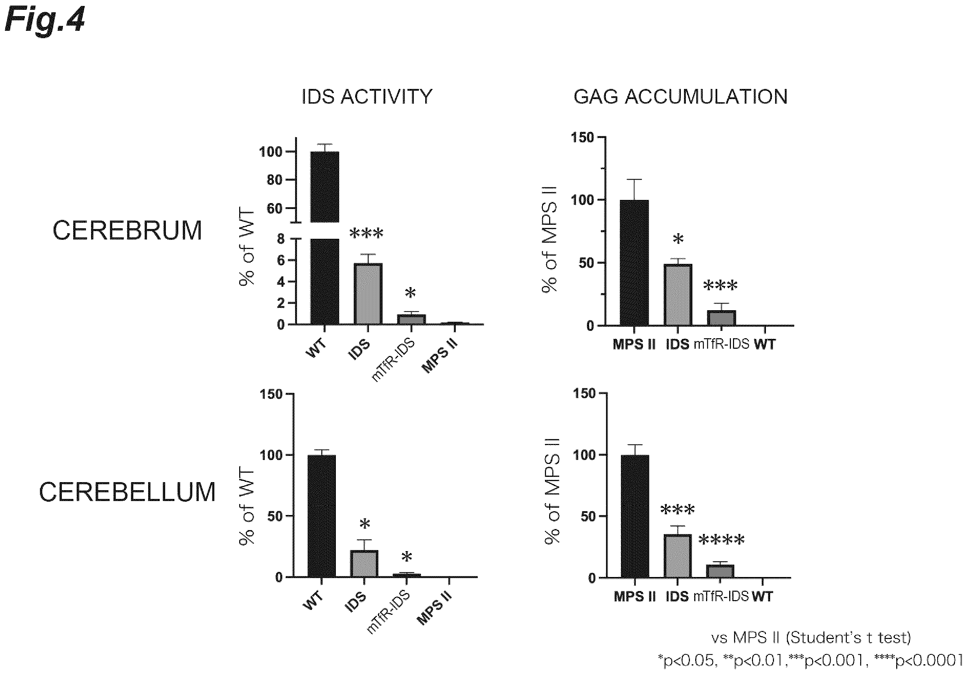

The results of measurement of the IDS activities and GAG accumulations in the cerebrum and cerebellum 6 months after transplantation are shown in FIG. 4. From FIG. 4, the mTfR-IDS mice showed low IDS activity, but an excellent reduction in GAG accumulation. In addition, the IDS activity in the cerebrum of the mTfR-IDS mice was about 1/6 that of the IDS mice and about 5 times higher than the activity ratio in the plasma. This fact suggested that, in the mTfR-IDS mice, IDS is secreted in the cerebrum and, additionally, IDS in the blood migrates to the cerebrum. Furthermore, the reduction rate of GAG accumulation in the cerebrum of the mTfR-IDS mice was higher than that of the IDS mice. These facts suggested that, due to the supply of IDS to the central nervous tissue of the mTfR-IDS mouse, IDS having migrated from the blood may be widely distributed in the tissue, in addition to the migration and local secretion of the cells derived from the transplanted hematopoietic stem cells to the central nerve.

From the above results, it is considered that the tissue distribution of IDS was improved by fusing the anti-mouse TfR antibody, resulting in an effect superior to that of the conventional IDS. A high blood IDS activity significantly exceeding that of the wild-type (WT) has hitherto been required in the treatment of central nervous lesions of MPS II, but it has been revealed that the mTfR-IDS mice can achieve superior therapeutic effects (reductions in GAG accumulations in the cerebrum and cerebellum) than those of the IDS mice with blood IDS activity comparable to that of the wild-type (WT) (see FIGS. 1 and 4).

### [Example 2: Hematopoietic Stem Cell Gene Therapy Using Anti-human TfR Antibody-Human IDS]

### <Mouse>

Female C57BL/6 mice (Higuchi T et al., Mol. Genet. Metab. 2012.; 107:122-128) having mouse IDS gene deficiency heterozygously and male knock-in mice (Sonoda H, et al, Mol Ther. 2018 May 2; 26 (5): 1366-1374) having a human transferrin receptor heterozygously were crossed, and mice (human transferrin receptor-KI/MPS II mice) having IDS deficiency hemizygously and hTfR heterozygously were selected from the created males by PCR analysis. C57BL/6 male mice served as normal controls.

### <Construction of Lentiviral Vector>

pJLV1-hTfR-IDS was constructed in which Fab-type anti-human TfR antibody-IDS (hTfR-IDS) was carried immediately below the MND promoter (DNA: SEQ ID NO: 1) of the pJLV1 plasmid (SEQ ID NO: 12).

### (1) Construction of pJLV1-hTfR-IDS

A vector pJLV1-hTfR-IDS (SEQ ID NO: 16) was produced by a cloning operation in which a DNA fragment (SEQ ID NO: 15) encoding a fusion protein (hTfR-IDS, SEQ ID NO: 14) in which the N-terminus of human IDS was functionally fused to the C-terminal side of the heavy chain of the Fab-type anti-human TfR antibody via a linker, and the N-terminus of the light chain of the Fab-type anti-human TfR antibody was functionally fused to the C-terminal side of human IDS via a self-cleaving peptide p2A was placed on the vector pJLV1.

Specifically, DNA encoding MND-hTfR-IDS was artificially synthesized in a state in which an MluI site was added to the 5' side and a XhoI site was added to the 3' side, and treated with a restriction enzyme. Next, the pJLV1 plasmid was also cleaved with MluI and XhoI, and pJLV1-hTfR-IDS (SEQ ID NO: 16) was produced using Quick ligation kit.

The lentiviral vector pJLV1-IDS prepared in "(1) Construction of pJLV1-IDS" in Example 1 was also used in Example 2.

### (2) Viral Packaging

As in Example 1, lentiviral packaging was performed using pCAG-HIVgp as a packaging plasmid and pCMV-VSV-G-RSV-Rev as a VSV-G/Rev plasmid, in addition to the self-inactivating (SIN) lentiviral vector (pJLV1-IDS or pJLV1-hTfR-IDS) produced above, by partially modifying a standard protocol (for example, the protocol described in Patent Literature 1).

The titer of the lentivirus prepared was measured using Quick Titer HIV Lentivirus Quantitation Kit (Cell Biolabs, San Diego, CA), which is an ELISA for p24 protein. The titers were: 1.6 × 10⁹ IU/ml for pJLV1-IDS and 1.7 × 10⁹ IU/ml for pJLV1-hTfR-IDS.

### <Production and Transplantation of Gene-introduced Hematopoietic Stem Cell>

Bone marrow cells were collected from the femur of 5-month-old human transferrin receptor-KI/MPS II mice, and mouse hematopoietic stem cells were separated using Lineage Cell Depletion Kit(Miltenyi Biotec, Bergisch Gladbach, Germany). The obtained lentivirus carrying IDS or hTfR-IDS was gene-introduced (infected) into the separated mouse hematopoietic stem cells. Lentiviral infection of the mouse hematopoietic stem cells was performed according to the method of the previous literature (Non Patent Literature 2) and at a multiplicity of infection (MOI = 50). Specifically, it was performed using 1 × 10⁸ IU of lentivirus per 2 × 10⁶ mouse hematopoietic stem cells (the number of cells for transplantation per recipient).

Transplantation of the gene-introduced hematopoietic stem cells was also performed according to the previously reported literature (Non Patent Literature 2). The gene-introduced hematopoietic stem cells were administered from the tail vein to the 5-month-old hTfR-KI/MPS II mice that had been irradiated with a lethal dose of 9 Gy using an X-ray irradiation system MBR-1520-R (Hitachi Power Solutions). Four weeks after administration, plasma, liver, spleen, cerebrum, and cerebellum were collected. Untransplanted wild-type (WT) C57BL/6 mice and human transferrin receptor -KI/MPS II mice (MPS II) were used as control mice.

### <IDS Activity Measurement and Glycosaminoglycan (GAG) Measurement>

The measurements of IDS activity and GAG were performed in the same manner as in Example 1.

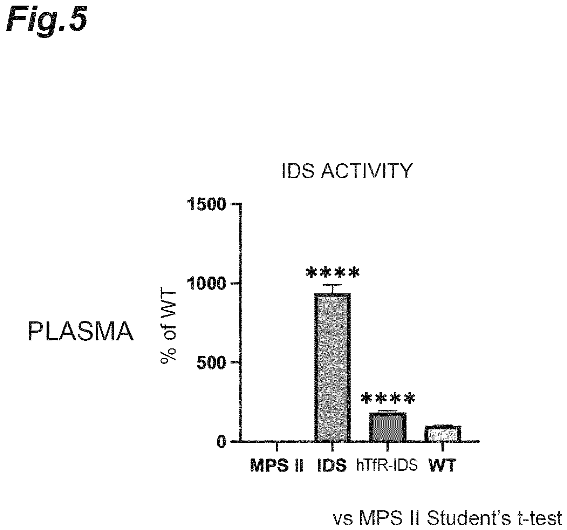

The IDS activity in the plasma 4 weeks after transplantation is shown in FIG. 5. The IDS activity in FIG. 5 is a relative activity (%) when the IDS activity in the wild-type mice (WT) is 100. From FIG. 5, both the IDS activities in the plasma of the IDS mice and the hTfR-IDS mice were higher than that of the wild-type mice, with the IDS activity of the IDS mice being 9.36 times higher than that of the wild-type mice and the IDS activity of the hTfR-IDS mice being 1.85 times higher than that of the wild-type mice.

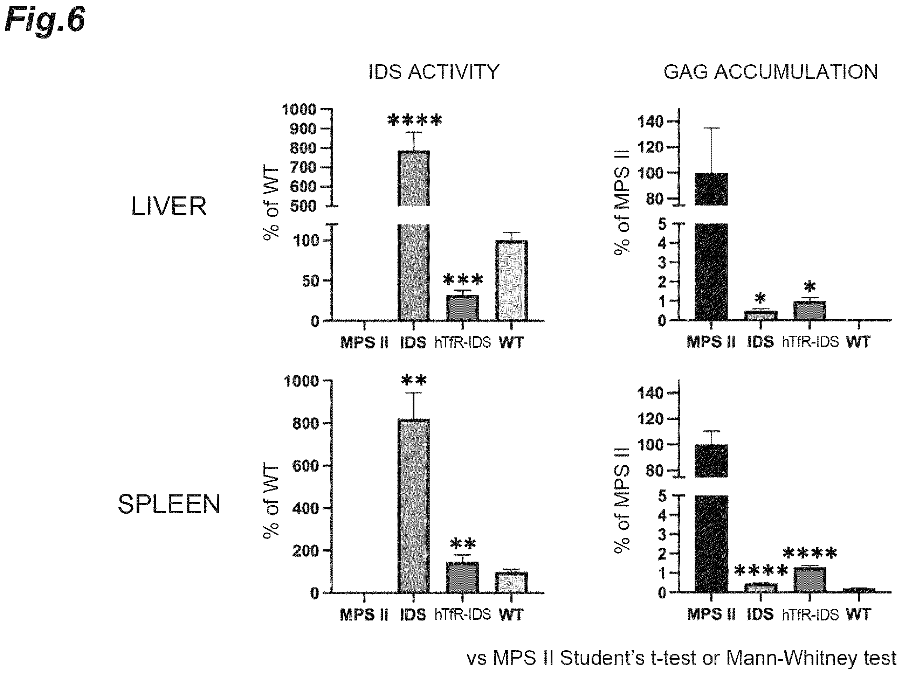

The results of measurement of the IDS activities and GAG accumulations in the liver and spleen 4 weeks after transplantation are shown in FIG. 6. The IDS activity is a relative activity (%) when the IDS activity in the wild-type mice (WT) is 100, and the GAG accumulation is a relative amount (%) when the amount of GAG accumulated in the human transferrin receptor-KI/MPS II mice (MPS II) is 100. From FIG. 6, the hTfR-IDS mice had IDS activity in the liver of 32.6% of that of the wild-type mice, IDS activity in the spleen of 147% of that of the wild-type mice, and a significant decrease in GAG accumulation was observed in both the organs.

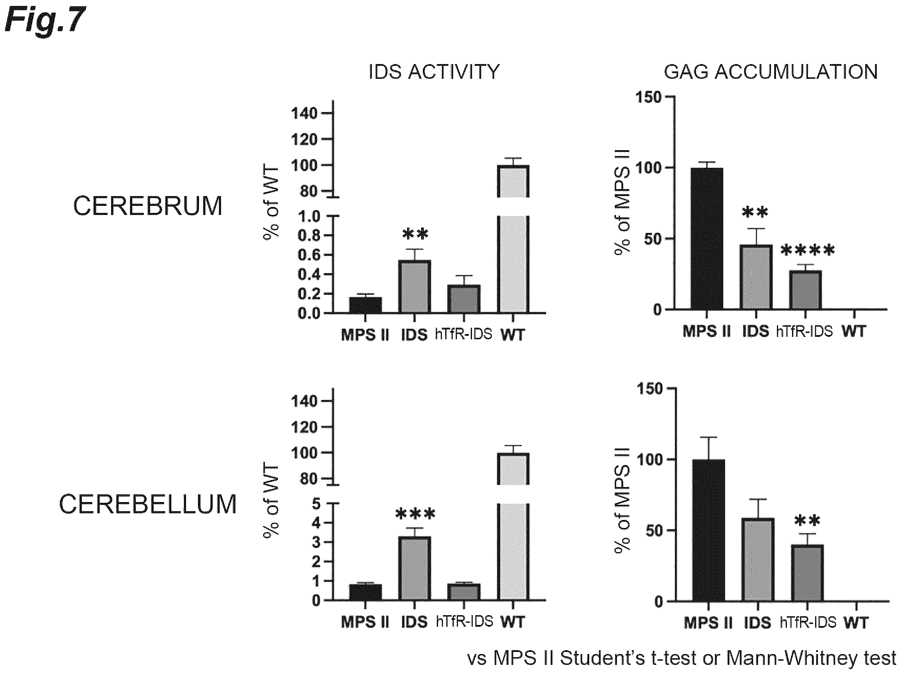

The results of measurement of the IDS activities and GAG accumulations in the cerebrum and cerebellum 4 weeks after transplantation are shown in FIG. 7. The IDS activity is a relative activity (%) when the IDS activity in the wild-type mice (WT) is 100, and the GAG accumulation is a relative amount (%) when the amount of GAG accumulated in the human transferrin receptor-KI/MPS II mice (MPS II) is 100. From FIG. 7, the hTfR-IDS mice showed low IDS activity, but an excellent reduction in GAG accumulation, as with the hTfR-IDS mouse of Example 1.

### [Example 3: T Cell Gene Therapy Using Anti-human TfR Antibody-Human IDS]

### <Mouse>

Mice similar to the mice obtained in Example 2 were used.

### <Construction of Lentiviral Vector>

Lentiviral packaging was performed using pCAG-HIVgp as a packaging plasmid and pCMV-VSV-G-RSV-Rev as a VSV-G/Rev plasmid, in addition to the self-inactivating (SIN) lentiviral vector (pJLV1-IDS or pJLV1-hTfR-IDS) produced in Example 2, by partially modifying a standard protocol (for example, the protocol described in Patent Literature 1).

### <Production and Transplantation of Gene-introduced T Cell>

Mouse T cells were separated using Pan T Cell Isolation Kit II (Miltenyi Biotec) from the spleen of 5-month-old human transferrin receptor-KI/MPS II mice. The obtained lentivirus carrying IDS or hTfR-IDS was gene-introduced (infected) into the separated mouse T cells. Lentiviral infection of the mouse T cells was performed at a multiplicity of infection (MOI) of 50 by applying stimulation for 24 hours according to the protocol of Dynabeads Mouse T-Activator CD3/CD28 (Thermo Fisher) followed by lentiviral infection. Specifically, it was performed using 5 × 10⁷ IU of lentivirus per 1 × 10⁶ mouse T cells (the number of cells for transplantation per recipient).

Regarding transplantation of the gene-introduced T cells, the 5-month-old human transferrin receptor-KI/MPS II mice were administered with the gene-introduced mouse T cells from the tail vein. Four weeks after administration, plasma, cerebrum, and cerebellum were collected. Untransplanted wild-type (WT) C57BL/6 mice and human transferrin receptor -KI/MPS II mice (MPS II) were used as control mice.

### <IDS Activity Measurement and Glycosaminoglycan (GAG) Measurement>

The measurements of IDS activity and GAG were performed in the same manner as in Example 1.

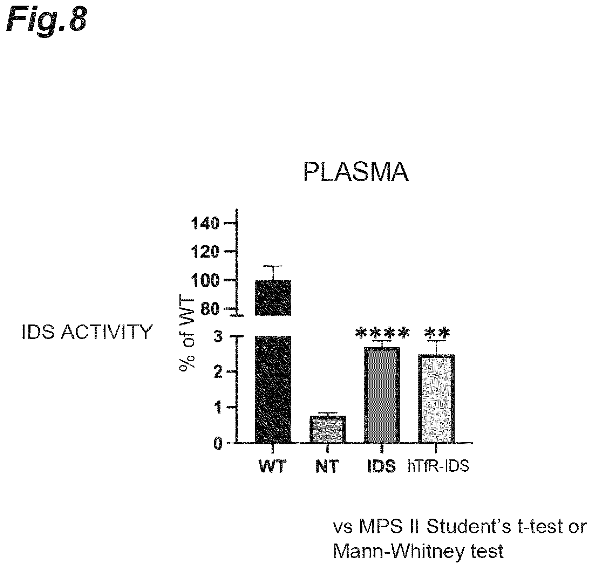

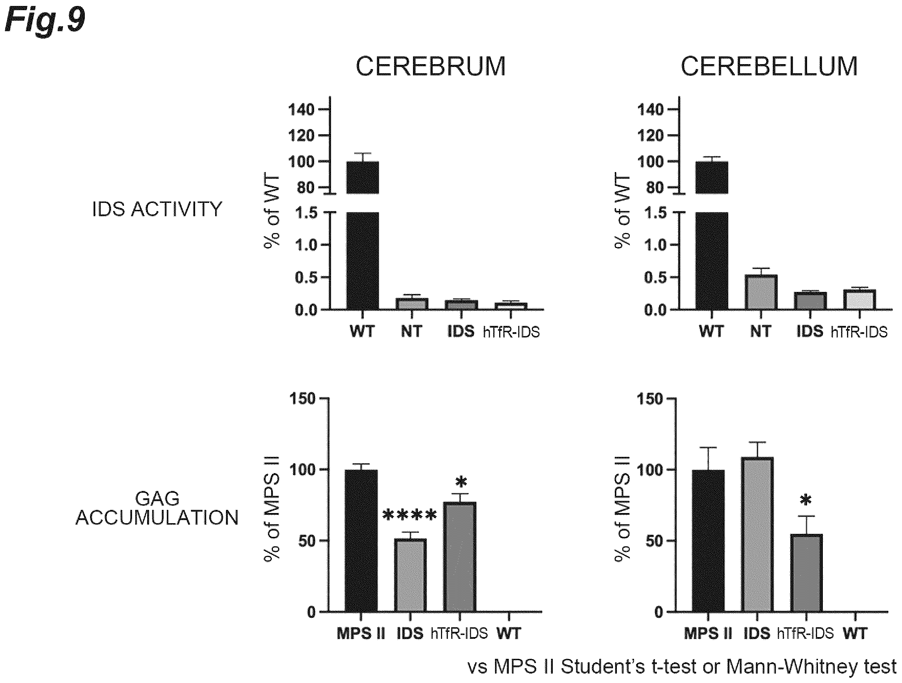

The results of measurement of the IDS activities in the plasma, cerebrum and cerebellum and GAG accumulations in the cerebrum and cerebellum 4 weeks after transplantation are shown in FIGS. 8 and 9. The IDS activity is a relative activity (%) when the IDS activity in the wild-type mice (WT) is 100, and the GAG accumulation is a relative amount (%) when the amount of GAG accumulated in the human transferrin receptor-KI/MPS II mice (MPS II) is 100. From FIG. 8, the IDS activities in the plasma of the IDS mice and the hTfR-IDS mice were both superior to that of the human transferrin receptor-KI/MPS II mice. From FIG. 9, even when mouse T cells were used, a decrease in GAG accumulation was observed in the cerebrum and cerebellum in the hTfR-IDS mice.

### [Example 4: Hematopoietic Stem Cell Gene Therapy Using Anti-mouse TfR Antibody-Human GAA]

### <Mouse>

Mice homozygously deficient in acid α glucosidase (GAA) gene were prepared by crossing (GAA-KO mice). C57BL/6 mice served as normal controls.

### <Construction of Lentiviral Vector>

pJLV1-GAA in which human GAA (wild-type human GAA, SEQ ID NO: 17) was carried immediately below the MND promoter (DNA: SEQ ID NO: 1) of the pJLV1 plasmid (SEQ ID NO: 12), which is a self-inactivating lentiviral vector, and pJLV1-mTfR-GAA in which anti-mouse TfR antibody-GAA (mTfR-GAA) was carried similarly immediately below the MND promoter were constructed.

### (1) Construction of pJLV1-GAA

DNA (SEQ ID NO: 18) encoding human GAA (SEQ ID NO: 17) was artificially synthesized in a state where a NotI site was added to the 5' side and a XhoI site was added to the 3' side, and treated with a restriction enzyme. Next, the pJLV1 plasmid (SEQ ID NO: 12) was also cleaved with NotI and XhoI, and pJLV1-GAA (SEQ ID NO: 19) was produced using Quick ligation kit.

### (2) Construction of pJLV1-mTfR-GAA

DNA (SEQ ID NO: 21) encoding a fusion protein (mTfR-GAA, SEQ ID NO: 20) in which human GAA (SEQ ID NO: 17) was functionally fused via a linker at the 3' terminus of an anti-mouse TfR antibody (anti-mouse TfR_scFV, SEQ ID NO: 6; codon optimized DNA: SEQ ID NO: 5) was artificially synthesized in a state where a NotI site was added to the 5' side and an XhoI site was added to the 3' side, and treated with a restriction enzyme. Next, the pJLV1 plasmid (SEQ ID NO: 12) was also cleaved with NotI and XhoI, and pJLV1-mTfR-GAA (SEQ ID NO: 22) was produced using Quick ligation kit.

### (3) Viral Packaging

As in Example 1, lentiviral packaging was performed using pCAG-HIVgp as a packaging plasmid and pCMV-VSV-G-RSV-Rev as a VSV-G/Rev plasmid, in addition to the self-inactivating (SIN) lentiviral vector (pJLV1-GAA or pJLV1-mTfR-GAA) produced in the above (1) or (2), by partially modifying a standard protocol (for example, the protocol described in Patent Literature 1).

The titer of the lentivirus prepared was measured using Quick Titer HIV Lentivirus Quantitation Kit (Cell Biolabs, San Diego, CA), which is an ELISA for p24 protein. The titers were: 1.7 × 10⁹ IU/ml for pJLV1-GAA and 1.2 × 10⁹ IU/ml for pJLV1-mTfR-GAA.

### <Evaluation of GAAActivity of mTfR-GAA Carried on Lentiviral Vector>

The lentivirus carrying GAA or mTfR-GAA was infected into 1 × 10⁵ HEK293T cells at an MOI of 10, **1,** or 0.1, and cultured for 24 hours. Thereafter, the medium was replaced with a lentivirus-free medium, the cells were further cultured for 48 hours, and then the medium and the cells were recovered. The GAA activity was measured for the cell extracts extracted with distilled water and the media. The GAA activity was measured as follows.

### <Measurement of GAAActivity>

For protein quantification, a DC protein assay kit (Bio-Rad) was used, and for GAA activity measurement, 4-methylumbelliferyl α-D-glucopyranoside (Sigma-Aldrich), which is a fluorescent substrate, was used according to the previously reported literature (Non Patent Literature 8).

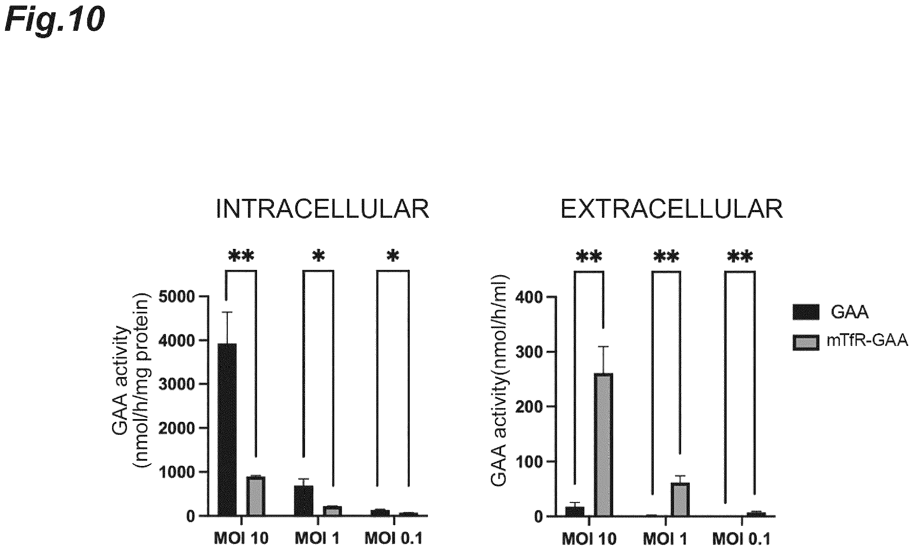

The results of measurement of the GAA activity for the HEK293T cell extracts and the media are shown in FIG. 10. From Figure 10, the intracellular activity was lower in the HEK293T cells infected with mTfR-GAA-carried lentivirus than that in the HEK293T cells infected with the GAA-carried lentivirus, while the extracellular activity (secreted enzyme activity) was significantly higher than that in the HEK293T cells infected with GAA-carried lentivirus. From the results, surprisingly, the extracellular secretion of GAA was promoted by fusing GAA with the anti-mouse TfR antibody. That is, it was found that fusing the anti-TfR antibody to the protein to be brought into function in the central nervous system not only improves the blood-brain barrier passability of the protein itself, but also brings about an effect of increasing the amount of the protein secreted to the outside of cells. Such an effect can be expected to dramatically enhance the effect of ex vivo gene therapy.

### <Production and Transplantation of Gene-introduced Hematopoietic Stem Cell>

In 3-month-old GAA-KO mice, Gene-introduced hematopoietic stem cells were prepared in the same procedures as in Example 1, and transplanted into the 3-month-old GAA-KO mice. The transplantation of the gene-introduced hematopoietic stem cells was also performed in the same manner as in Example 1 except that the serum, liver, spleen, heart, quadriceps femoris, diaphragm, gastrocnemius muscle, cerebrum, and cerebellum were collected 4 weeks after administration, and that untransplanted wild type (WT) C57BL/6 mice and GAA-KO mice, and GAA-KO mice (ERT) administered with recombinant GAA protein (avalglucosidase alpha (Sanofi)) at 20 mg/kg once every 2 weeks from the tail vein were used as control mice.

### <GAAActivity Measurement and Glycogen Measurement>

Protein quantification was performed using a DC protein assay kit (Bio-Rad), and GAA activity measurement was performed as described above. The glycogen measurement was performed by the following method using Glycogen Assay Kit (abcam). In the Glycogen Assay Kit (abcam), glycogen is first hydrolyzed to glucose, an oxidized intermediate is then labeled with OxiRed Probe, and fluorescence is measured with Synergy H1 (BioTek) to quantify the glycogen. First, 50 µL each of a standard sample for a calibration curve containing glycogen at a known concentration and a tissue homogenate supernatant are added to 96F Nontreated Black microwell (Thermo scientific). Next, 1 µL of Hydrolysis Enzyme Mix as a hydrolysis reaction reagent is added to each well and mixed, and the mixture is reacted at room temperature for 30 minutes (however, the reagent is not added to wells for measuring endogenous glucose). Next, 50 µL of Development Enzyme Mix containing OxiRed Probe was added to each well and mixed, the mixture is reacted at room temperature for 30 minutes in the dark, and measurement was performed at an excitation wavelength of 535 nm and a detection wavelength of 587 nm using Synergy H1 (BioTek).

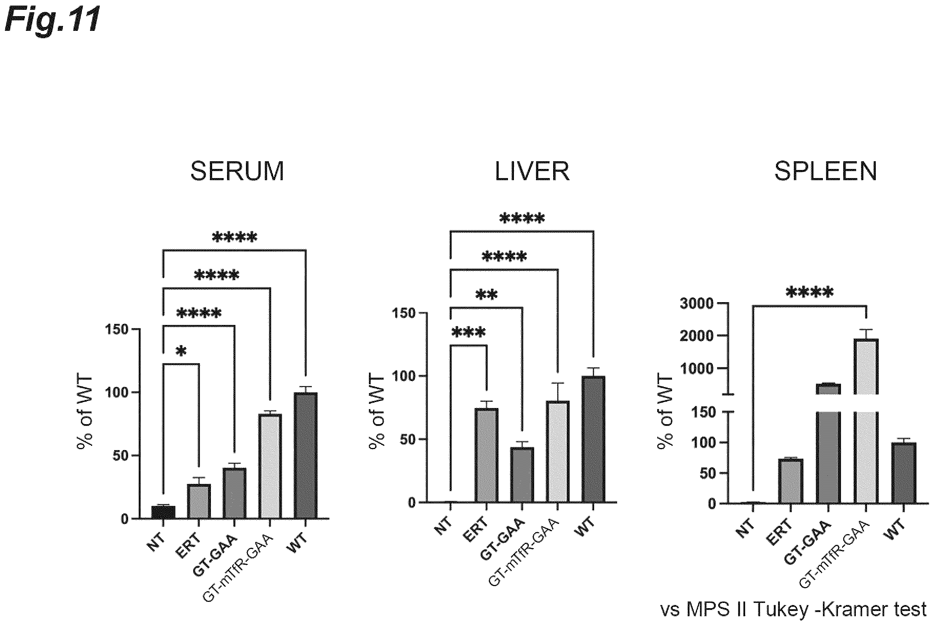

The results of measurement of the GAA activities in the serum, liver and spleen 4 weeks after transplantation are shown in FIG. 11. The GAA activity is a relative activity (%) when the GAA activity in the wild-type mice (WT) is 100. From FIG. 11, when the GAA activities in the serum, liver, and spleen were compared among the treatment groups (ERT mice, GAA mice, mTfR-GAA mice), the mTfR-GAA mice were the highest in all cases.

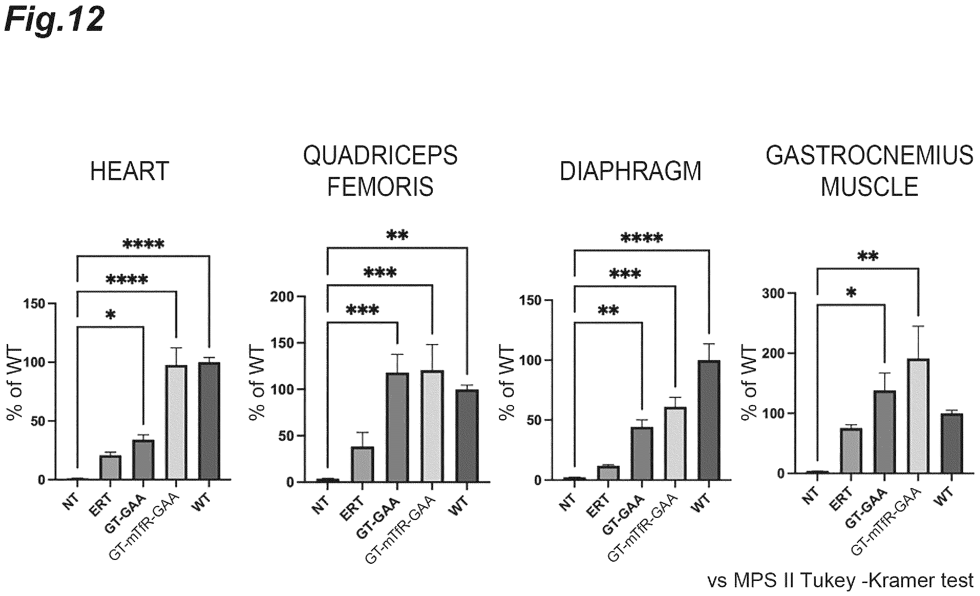

FIG. 12 shows results of measurement of the GAA activities in the heart, quadriceps femoris, diaphragm, and gastrocnemius muscle 4 weeks after transplantation. The GAA activity is a relative activity (%) when the GAA activity in the wild-type mice (WT) is 100. From FIG. 12, when the GAA activities in muscles such as the heart, quadriceps femoris, diaphragm, and gastrocnemius muscle were compared among the treatment groups, the mTfR-GAA mouse was the highest in all muscles. In addition, in the heart, quadriceps femoris, and gastrocnemius muscle, the GAA activity equal to or higher than that of the wild-type mice was observed. The GAA mice also showed the second highest GAA activity after the mTfR-GAA mice, and showed GAA activities in the quadriceps femoris and gastrocnemius muscle equal to or higher than those of the wild-type mice.

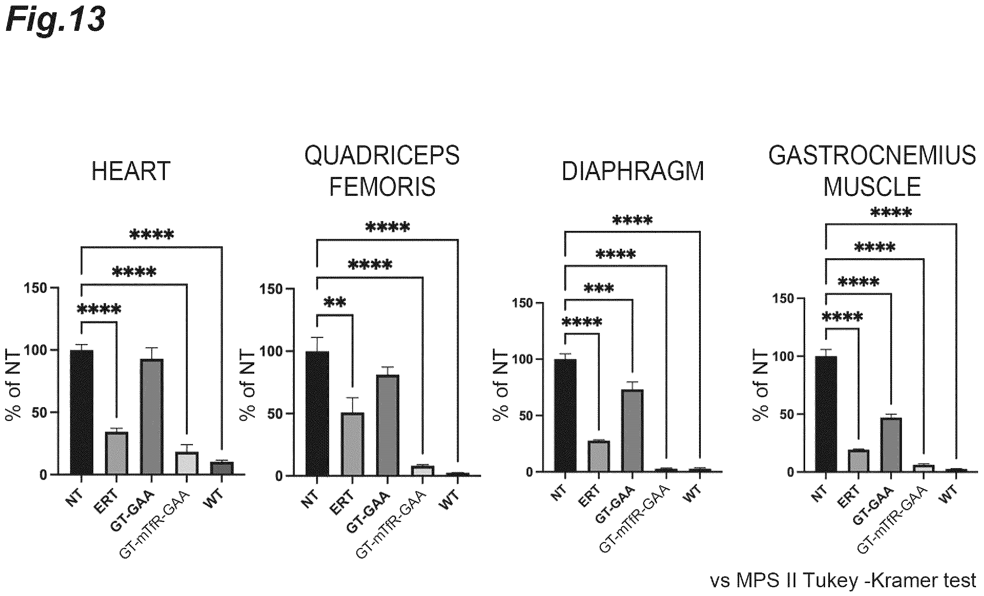

FIG. 13 shows results of measurement of the glycogen accumulations in the heart, quadriceps femoris, diaphragm, and gastrocnemius muscle 4 weeks after transplantation. The glycogen accumulation is a relative amount (%) when the amount of glycogen accumulated in GAA-KO mice (NT) is 100. From FIG. 13, the mTfR-GAA group achieved dramatic improvement in glycogen accumulation in various muscle tissues, and showed normalization thereof in the diaphragm. On the other hand, the GAA mice showed GAA activities in the quadriceps femoris and the like comparable to those of mTfR-GAA, but showed the least decrease in glycogen accumulation among the treatment groups. This suggested that the fusion of the anti-TfR antibody improved the distribution and localization of GAA.

In Pompe disease, it is known that resistance is exhibited due to occurrence of an abnormality called autophagic buildup in muscle tissue to cause abnormal localization of a therapeutic enzyme (for example, GAA). The reason why the glycogen decreasing effect is low in the GAA mice despite the relatively high enzyme activity of GAA is considered to be the influence of the abnormal localization of GAA described above. The mTfR-GAA is also expected to accumulate to cause autophagic buildup after being taken into cells, but surprisingly, glycogen accumulation was dramatically reduced in the mTfR-GAA mice. This is a surprising result.

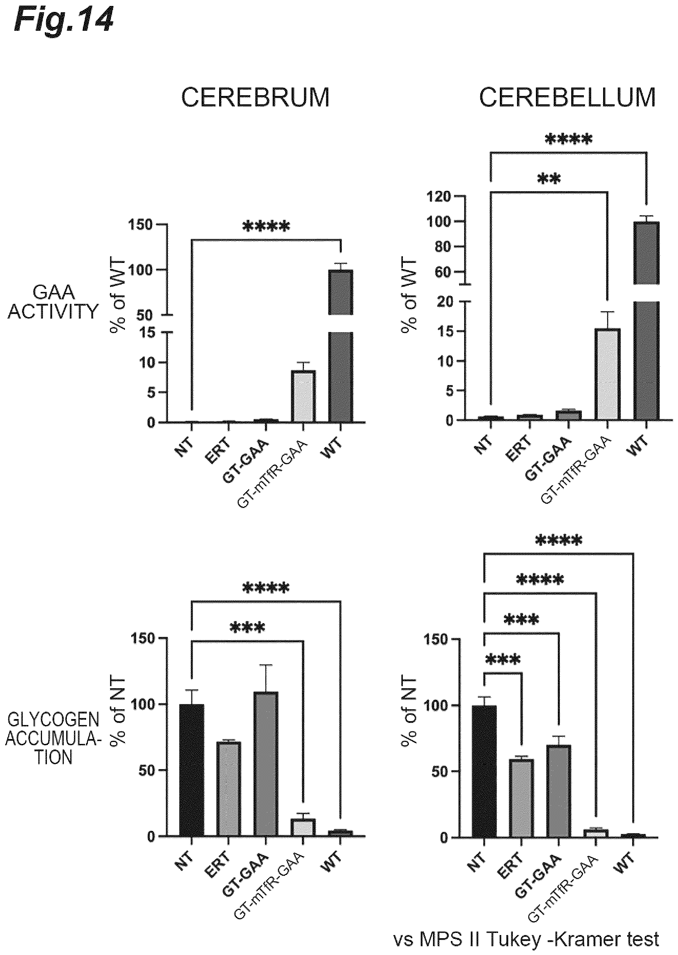

The results of measurement of the GAA activities and glycogen accumulations in the cerebrum and cerebellum 4 weeks after transplantation are shown in FIG. 14. The GAA activity is a relative activity (%) when the GAA activity in the wild-type mice (WT) is 100, and the glycogen accumulation is a relative amount (%) when the amount of glycogen accumulated in GAA-KO mice (NT) is 100. From FIG. 14, an increase in GAA activity was observed only in the mTfR-GAA mice and the glycogen accumulation was also significantly reduced.

### <Evaluation ofAnti-GAAAntibody Titer in Serum in Mouse after Treatment>

Measurement of anti-GAA antibody titer in the serum of the GAA-KO mice after gene therapy using gene-introduced hematopoietic stem cells was performed by partially modifying ELISA in a previously reported literature (Non Patent Literature 10). ELISA was performed by the following procedures. To a microplate (Maxisorp, Thermo Scientific), 250 ng of recombinant GAA protein (avalglucosidase alpha (Sanofi)) was bound overnight at 4°C, and then blocking was performed with PBS containing 1% BSA for 2 hours. Each well was washed using PBS containing 0.05% Tween 20 (PBS-T), then an anti-human GAA antibody (Proteintech) was added to the serum of each mouse or for preparing a calibration curve, and the mixture was reacted at normal temperature for 1 hour. After washing each well with PBS-T, a peroxidase-labeled anti-mouse IgG antibody (Histofine Simple Stain MAX-PO (M), Nichirei Bioscience Inc.) was added to the well to which each mouse serum was added, and a peroxidase-labeled anti-rabbit IgG antibody (Histofine Simple Stain MAX-PO (R), Nichirei Bioscience Inc.) was added to the well for preparing a calibration curve, and the mixture was reacted at normal temperature for 1 hour. Thereafter, each well was washed with PBS-T, tetramethylbenzidine (SeraCare) was added thereto to develop color for 10 minutes, and then 1.2 N sulfuric acid was added thereto to stop the reaction. For the measurement, the absorbance was measured at 450 nm and 650 nm using Synergy H1 (BioTek), and the difference therebetween was taken as true absorbance. The results are shown in Table 1.

**[Table 1]**

| Mouse | Antibody titer |
|---|---|
| NT (untreated mouse) | 23.9 ng/ml ± 15.2 |
| WT (wild-type mouse) | 21.0 ng/ml ± 8.6 |
| ERT mouse (ERT) | 443649 ng/ml ± 220683 |
| GAA mouse (GT-GAA) | 167.4 ng/ml ± 92.49 |
| mTfR-GAA mouse (GT-mTfR-GAA) | 32.9 ng/ml ± 25.8 |

In the ERT mice treated with ERT, the antibody titer of the anti-GAA antibody in the serum after treatment was the highest. In addition, in the GAA mice subjected to gene therapy using GAA introduced hematopoietic stem cells, the antibody titer of the anti-GAA antibody in the serum after treatment was lower than that in the ERT mice. Such results were consistent with the facts that the anti-GAA antibody is often produced during treatment of ERT, which is the current standard therapy, to diminish the therapeutic effect, that there are cases in which it is difficult to continue treatment due to allergic reactions, and that it is unlikely to produce antibodies in hematopoietic stem cell gene therapy of other diseases. On the other hand, unexpectedly, in the mTfR-GAA mice subjected to gene therapy using mTfR-GAA introduced hematopoietic stem cells, the antibody titer of the anti-GAA antibody was the lowest, and was at the same level as those of the untreated mice and the wild-type mice.

### [Example 5: Hematopoietic Stem Cell Gene Therapy Using Anti-mouse TfR Antibody-Human GLB1]

### <Mouse>

Mice heterozygously deficient in β-galactosidase (GLB1) gene (Non Patent Literature 9) were prepared by crossing (GLB1-KO mice). C57BL/6 mice served as normal controls.

### <Construction of Lentiviral Vector>

pJLV1-GLB1 in which human GLB1 (wild-type human GLB1, SEQ ID NO: 23) was carried immediately below the MND promoter (DNA: SEQ ID NO: 1) of the pJLV1 plasmid (SEQ ID NO: 12), which is a self-inactivating lentiviral vector, and pJLV1-mTfR-GLB1 in which anti-mouse TfR antibody-GLB1 (mTfR-GLB1) was carried similarly immediately below the MND promoter were constructed.

### (1) Construction of pJLV1-GLB1

DNA (SEQ ID NO: 24) encoding human GLB1 (SEQ ID NO: 23) was placed downstream of the MND promoter and prepared by artificial synthesis. Specifically, MND-GLB1 in which an MluI site was added to the 5' side and a XhoI site was added to the 3' side was artificially synthesized, and treated with a restriction enzyme. Next, the pJLV1 plasmid (SEQ ID NO: 12) was also cleaved with MluII and XhoI, and pJLV1-GLB1 (SEQ ID NO: 25) was produced using Quick ligation kit.

### (2) Construction of pJLV1-mTfR-GLB1

DNA (SEQ ID NO: 27) encoding a fusion protein (mTfR-GLB1, SEQ ID NO: 26) in which human GLB1 (SEQ ID NO: 23) was functionally fused via a linker at the 3' terminus of an anti-mouse TfR antibody (anti-mouse TfR_scFV, SEQ ID NO: 6; codon optimized DNA: SEQ ID NO: 5) was placed downstream of the MND promoter and prepared by artificial synthesis. Specifically, MND-mTfR-GLB1 in which an MluI site was added to the 5' side and a XhoI site was added to the 3' side was artificially synthesized, and treated with a restriction enzyme. Next, the pJLV1 plasmid (SEQ ID NO: 12) was also cleaved with MluII and XhoI, and pJLV1-mTfR-GLB1 (SEQ ID NO: 28) was produced using Quick ligation kit.

### (3) Viral Packaging

As in Example 1, lentiviral packaging was performed using pCAG-HIVgp as a packaging plasmid and pCMV-VSV-G-RSV-Rev as a VSV-G/Rev plasmid, in addition to the self-inactivating (SIN) lentiviral vector (pJLV1-GLB1 or pJLV1-mTfR-GLB1) produced in the above (1) or (2), by partially modifying a standard protocol (for example, the protocol described in Patent Literature 1).

The titer of the lentivirus prepared was measured using Quick Titer HIV Lentivirus Quantitation Kit (Cell Biolabs, San Diego, CA), which is an ELISA for p24 protein. The titers were: 1.8 × 10⁸ IU/ml for pJLV1-GLB1 and 4.3 × 10⁸ IU/ml for pJLV1-mTfR-GLB1.

### <Production and Transplantation of Gene-introduced Hematopoietic Stem Cell>

In 10-week-old GLB1-KO mice, gene-introduced hematopoietic stem cells were prepared in the same procedures as in Example 1, and transplanted into the 10-week-old GLB1-KO mice.

### <GLB1 Activity Measurement and GM1 Ganglioside Measurement>

Protein quantification was performed using a DC protein assay kit (Bio-Rad), GLB1 activity measurement was performed using 4-methylumbelliferyl-β-D-galactopyranoside, (Sigma-Aldrich) as a fluorescent substrate in accordance with the previously reported document (Non-Patent Document 7), and GM1 ganglioside measurement was also performed using a triple quadrupole high-performance liquid chromatograph mass spectrometer LCMS-8040 (Shimadzu Corporation) in accordance with the previously reported document (Non Patent Literature 7).

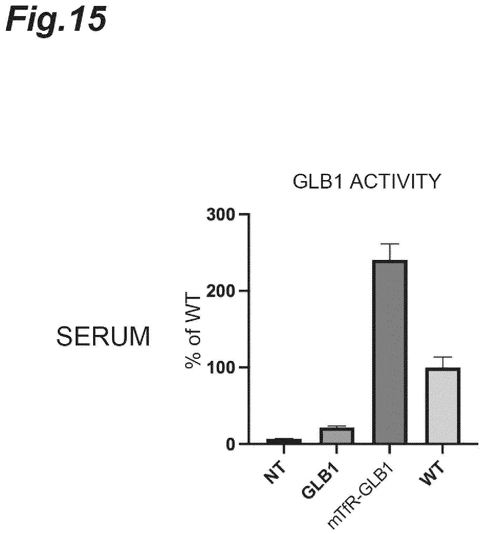

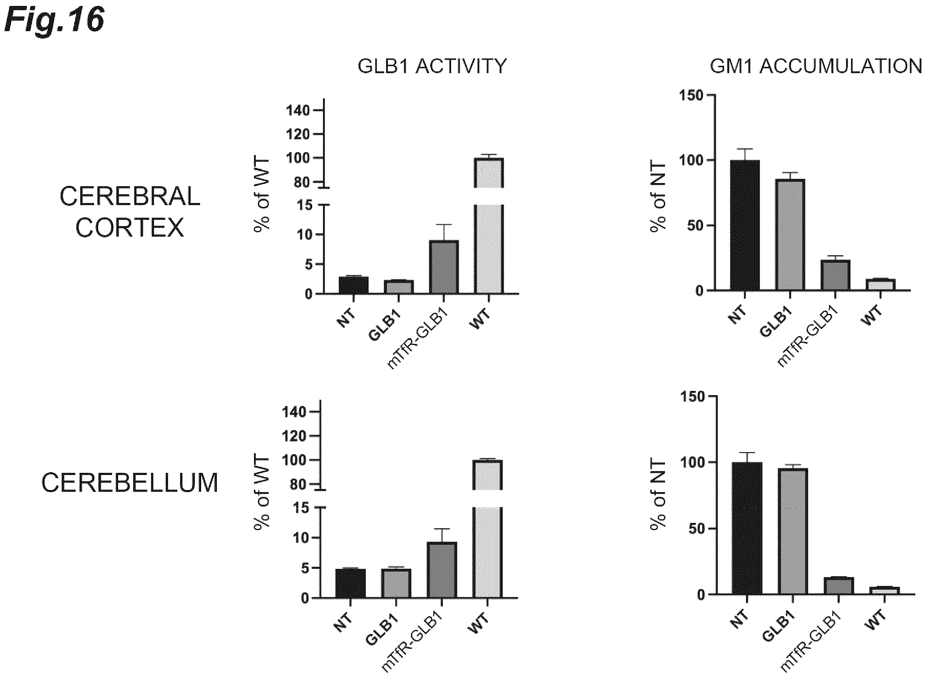

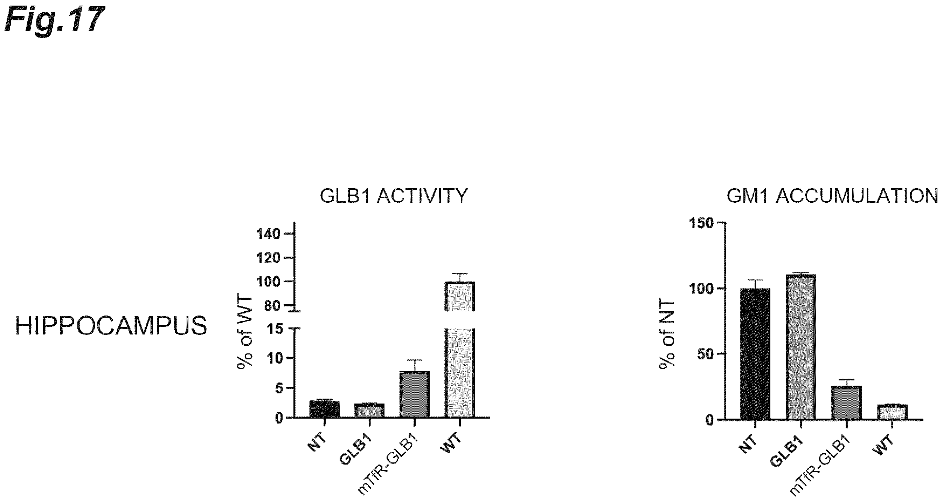

FIGS. 15 to 17 show measurement results of GLB1 activities and GM1 ganglioside accumulations (GM1 accumulations) in the serum, cerebral cortex, cerebellum, and hippocampus 4 weeks after transplantation. The GLB1 activity is a relative activity (%) when the GLB1 activity in the wild-type mice (WT) is 100, and the GM1 accumulation is a relative amount (%) when the amount of GM1 accumulated in GLB1-KO mice (NT) is 100. From FIGS. 16 to 17, the mTfR-GLB1 mice showed high GLB1 activities in all of the cerebral cortex, cerebellum, and hippocampus, and showed significantly reduced GM1 accumulations therein.

In general, it is considered that the activity of the fusion protein is lower than the activity of the protein that does not form the fusion protein due to fusion of the anti-mTfR antibody, but, unexpectedly, the anti-mTfR antibody fusion protein showed higher blood GLB 1 activity than that of the wild-type enzyme. It is considered that the increase in amount of extracellular secretion and stabilizing action of the enzyme in the blood, which would have been caused by the fusion of the anti-mTfR antibody, may exceed the decrease in activity. Since GAA and GLB 1 are originally poorly secreted or unstable enzymes, the blood activity was not reduced but rather increased by fusion of the anti-mTfR antibody, which was an unexpected result.

## Claims

1. A nucleic acid molecule comprising a nucleotide sequence encoding a fusion protein, the fusion protein comprising: an anti-transferrin receptor antibody or an antigen-binding fragment thereof; and a protein to be brought into function in a central nervous system.

2. The nucleic acid molecule according to claim **1,** wherein the protein to be brought into function in the central nervous system is a lysosomal enzyme.

3. The nucleic acid molecule according to claim 2, wherein the lysosomal enzyme is acid α-glucosidase or β-galactosidase.

4. The nucleic acid molecule according to claim 1, wherein the protein to be brought into function in the central nervous system is iduronate-2-sulfatase.

5. The nucleic acid molecule according to claim **1,** wherein the protein to be brought into function in the central nervous system is a neurotrophic factor.

6. The nucleic acid molecule according to claim **1,** wherein the protein to be brought into function in the central nervous system is an antibody.

7. A vector comprising the nucleic acid molecule according to any one of claims 1 to 6.

8. The vector according to claim 7, wherein the vector stably expresses the fusion protein when the nucleic acid molecule is introduced into a host cell using the vector.

9. The vector according to claim 8, wherein the vector is a lentiviral vector.

10. A recombinant cell obtained by introducing the nucleic acid molecule into a host cell using the vector according to claim 7.

11. The recombinant cell according to claim 10, wherein the host cell is a hematopoietic stem cell, a T cell, or a B cell.

12. A drug for diagnosing, preventing or treating a central nervous system disease, the drug comprising the recombinant cell according to claim 10.

13. A method for diagnosing, preventing, or treating a central nervous system disease, the method comprising transplanting the recombinant cell according to claim 10 into a subject in need thereof.
